# FASCICULE DE BREVET EUROPEEN

(11) **EP 0 934 244 B1**
(45) Date de publication et mention de la délivrance du brevet: **15.12.2004**
(21) Numéro de dépôt: 97909401.8
(22) Date de dépôt: 13.10.1997
(51) Int. Cl.: C07C 65/05, C07C 51/255, C07C 47/565, C07C 45/38

(54) **PROCEDE DE PREPARATION SELECTIVE D'UN ACIDE 2-HYDROXYBENZOIQUE ET D'UN 4-HYDROXYBENZALDEHYDE ET DERIVES**
VERFAHREN ZUR SELEKTIVEN HERSTELLUNG VON 2-HYDROXYBENZOESÄURE UND VON 4-HYDROXYBENZALDEHYD UND DERIVATEN
METHOD FOR SELECTIVE PREPARATION OF A 2-HYDROXYBENZOIC ACID AND A 4-HYDROXYBENZALDEHYDE AND DERIVATIVES

(30) Priorité: 14.10.1996 FR 9612479
(43) Date de publication de la demande: 11.08.1999
(73) Titulaire: RHODIA CHIMIE, 92408 Courbevoie Cédex (FR)
(72) Inventeur: METIVIER, Pascal, F-69110 Sainte Foy les Lyon (FR); MALIVERNEY, Christian, F-69007 Lyon (FR); DENIS, Philippe, F-69150 Décines (FR)
(74) Mandataire: Dutruc-Rosset, Marie-Claude
(86) Numéro de dépôt international: PCT/FR1997/001828
(87) Numéro de publication internationale: WO 1998/016493

(56) Documents cités:
- EP-A- 0 000 165
- EP-A- 0 667 331
- WO-A-96/37454
- DE-B- 1 188 069
- US-A- 4 351 962

## Description

La présente invention a pour objet un procédé de préparation d'un acide 2-hydroxybenzoïque et d'un 4-hydroxybenzaldéhyde et dérivés, à partir d'un mélange de deux composés phénoliques, l'un portant un groupe formyle ou hydroxyméthyle en position 2 et l'autre portant un groupe formyle ou hydroxyméthyle en position 4.

Un autre objet de l'invention est la préparation d'un 4-hydroxybenzaldéhyde à partir dudit mélange.

L'invention concerne plus particulièrement la préparation du 3-méthoxy-4-hydroxybenzaldéhyde et du 3-éthoxy-4-hydroxybenzaldéhyde dénommés respectivement "vanilline" et "éthylvanilline".

II est connu selon US 4 351 962 de préparer des hydroxybenzaldéhydes par réaction d'un composé phénolique et du formaldéhyde, en présence d'une base permettant d'obtenir un composé phénolique mono- ou polyhydroxyméthylé, oxydation par l'oxygène, en milieu basique, en présence de palladium ou de platine, puis décarbonylation du groupe formyle indésirable.

Ainsi, la vanilline est préparée par réaction du gaïacol et du formaldéhyde, en présence de soude, oxydation par l'oxygène, en présence de soude et d'un catalyseur à base de palladium puis décarbonylation du groupe formyle situé en position ortho du groupe hydroxyle.

Selon US 4 351 962, la quantité de base mise en oeuvre au cours de l'oxydation est définie comme étant ajustée à au moins 10 % du rapport molaire pénol/base de 1:1 avec une limite supérieure de 1:2 mais choisi préférentiellement à l'intérieur d'une gamme allant de 1 : 1,1 à 1 : 2.

Dans la demande de brevet française n°95/06186, on a décrit un procédé de préparation de 4-hydroxybenzaldéhydes et plus particulièrement de vanilline et d'éthylvanilline.

Le procédé décrit consiste à préparer un 3-carboxy-4-hydroxybenzaldéhyde puis à effectuer la décarboxylation dudit composé conduisant ainsi au 4-hydroxybenzaldéhyde.

Le 3-carboxy-4-hydroxybenzaldéhyde est préparé selon FR n°95/06186, à partir de l'un des composés et leurs mélanges répondant plus particulièrement aux formules suivantes (IIa), (IIb), (IIc) et (IId) données ci-aprés : 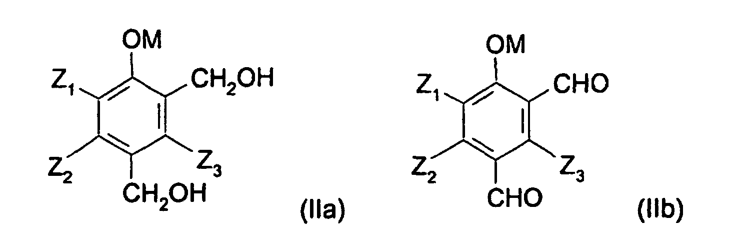 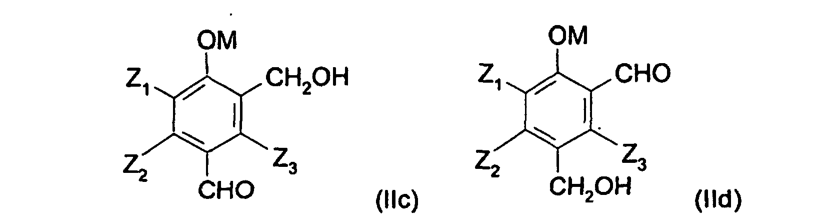 dans lesdites formules :
- M représente un atome d'hydrogène et/ou un cation métallique du groupe (la) ou (IIa) ou un cation ammonium,
- Z₁, Z₂ et Z₃, identiques ou différents, représentent un atome d'hydrogène, un radical alkyle, alcényle, alkoxy, hydroxyalkyle, alkoxyalkyle, cycloalkyle, aryle, un groupe hydroxyle, un groupe nitro, un atome d'halogène, un groupe trifluorométhyle.

Selon le procédé breveté, on part d'un composé phénolique bi-fonctionnel, portant sur le noyau aromatique, deux groupes fonctionnels en ortho et para du groupe hydroxyle qui peuvent être un groupe - CHO et/ou un groupe - CH₂OH.

On effectue d'abord une oxydation sélective du groupe situé en position ortho en groupe carboxylique ; le groupe situé en position para étant au plus oxydé en groupe formyle. Ainsi, après élimination du groupe carboxylique situé en position ortho, on obtient un 4-hydroxybenzaldéhyde.

On obtient avantageusement de la vanilline et de l'éthyivanilline selon un procédé sélectif mais aussi très compétitif d'un point de vue industriei car il fait appel à des reactifs peu onéreux.

Poursuivant ses recherches, la demanderesse a trouvé qu'il était possible de partir d'un mélange de composes phénoliques monosubstitués.

Ainsi, il a été trouvé et c'est ce qui constitue l'objet de la présente invention, un procédé de préparation d'un acide 2-hydroxybenzoïque et d'un 4-hydroxybenzaldéhyde et dérivés qui est caractérisé par le fait que l'on soumet un mélange de composés phénoliques, l'un (A) porteur d'un groupe formyle ou hydroxyméthyle en position 2, et l'autre (B) porteur d'un groupe formyle ou hydroxyméthyle en position 4, à une oxydation sélective du groupe formyle ou hydroxyméthyie en position 2 du composé (A) en groupe carboxyiique, et éventuellement d'un groupe hydroxyméthyle du composé (B) en position 4 en groupe formyie, conduisant ainsi à un mélange d'un acide 2-hydroxybenzoïque et d'un 4-hydroxybenzaldéhyde.

Dans une étape successive, on sépare le 4-hydroxybenzaldéhyde du milieu réactionnel.

Une première variante de l'invention consiste à séparer le 4-hydroxybenzaldéhyde de l'acide 2-hydroxybenzoïque, par extraction de l'aldéhyde à pH contrôlé.

Une autre variante de l'invention consiste à soumettre le mélange obtenu à une décarboxylation uniquement de l'acide 2-hydroxybenzoïque permettant d'aboutir au composé phénolique de départ qui peut être alors recyclé ; le 4-hydroxybenzaldéhyde étant alors récupéré d'une manière classique.

Conformément à la présente invention, il a été trouvé que lorsque l'on partait d'un mélange de molécules phénoliques, l'une portant des groupes hydroxyméthyle ou formyle en position ortho du groupe hydroxyle et l'autre portant un groupe hydroxyméthyle ou formyle en position para du groupe hydroxyle, l'oxydation en groupe carboxylique a lieu préférentiellement sur le groupe hydroxyméthyle ou formyle porté par la molécule (A), substituée en position ortho.

Les substrats de départ intervenant dans le procédé de l'invention sont des mélanges de composés phénoliques l'un porteur d'un groupe formyle ou hydroxyméthyle en position 2 et l'autre, en position 4.

Par "composé phénolique", on entend tout composé aromatique, dont le noyau aromatique est porteur d'un groupe hydroxyle.

Dans l'exposé qui suit de la présente invention, on entend par "aromatique", la notion classique d'aromaticité telle que définie dans la littérature, notamment par Jerry MARCH, Advanced Organic Chemistry, 4^{ème} édition, John Wiley and Sons, 1992, pp. 40 et suivantes.

Ainsi, on met en oeuvre un mélange (II) de composés phénoliques répondant plus particulièrement aux formules suivantes : 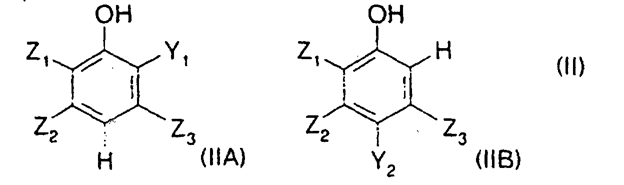 dans lesdites formules (IIA) et (IIB) :
- Y₁ et Y₂, identiques ou différents, représentent l'un des groupes suivants :
   . un groupe - CHO,
   . un groupe - CH₂OH,
- Z₁, Z₂ et Z₃, identiques ou différents, représentent un atome d'hydrogène, un radical alkyle, alcényle, alkoxy, hydroxyalkyle, alkoxyalkyle, cycloalkyle, aryle, un groupe hydroxyle, un groupe nitro, un atome d'halogène, un groupe trifluorométhyle.

Les composés qui conviennent particulièrement bien à la mise en oeuvre du procédé de l'invention répondent aux formules (IIA) et (IIB) dans lesquelles Z₁, Z₂ et Z₃, identiques ou différents, représentent l'un des atomes ou groupes suivants :
. un atome d'hydrogène.
. un radical alkyle, linéaire ou ramifié, ayant de 1 à 12 atomes de carbone, de préférence de 1 à 4 atomes de carbone, tel que méthyle, éthyle, propyle, isopropyle, butyle, isobutyle, sec-butyle, tert-butyle,
. un radical alcényle, linéaire ou ramifié, ayant de 2 à 12 atomes de carbone, de préférence de 2 à 4 atomes de carbone, tel que vinyle, allyle,
. un radical alkoxy linéaire ou ramifié ayant de 1 à 12 atomes de carbone, de préférence de 1 à 4 atomes de carbone tel que les radicaux méthoxy, éthoxy, propoxy, isopropoxy, butoxy, isobutoxy, sec-butoxy, tert-butoxy,
. un radical phényle,
. un atome d'halogène, de préférence un atome de fluor, chlore ou brome.

La présente invention n'exclut pas la présence sur le cycle aromatique de substituants d'une nature différente dans la mesure où ils n'interfèrent pas avec les réactions du procédé de l'invention.

La présente invention s'applique préférentiellement aux composés de formule (IIA) et (IIB) dans lesquelles Z₁ représente un atome d'hydrogène ou un radical alkyle ou alkoxy, linéaire ou ramifié, ayant de 1 à 6 atomes de carbone, de préférence, 1 à 4 atomes de carbone ; Z₂ et Z₃ représentant un atome d'hydrogène ; Y₁ et Y₂, identiques, représentent un groupe formyle ou un groupe hydroxyméthyle.

Comme exemples préférés de mélangés de composés phénoliques susceptibles d'être mis en oeuvre dans le procédé de l'invention, on peut citer, entre autres :
- l'o-hydroxyméthylphénol et le p-hydroxyméthylphénol,
- l'o-hydroxyméthylgaïacol et le p-hydroxyméthylgaïacol,
- l'o-formylgaïacol et le p-formylgaïacol.
- l'o-hydroxyméthylguétol et le p-hydroxyméthylguétol,
- l'o-formylguétol et le p-formylguétol.

Conformément au procédé de l'invention, on part d'un mélange de composés phénoliques répondant préférentiellement à la formule (II).

La proportion dans le mélange de chaque composé phénolique hydroxyméthylé ou formylé dépend du mode de leur préparation.

On précisera à titre indicatif que la proportion de chaque isomère peut varier largement, par exemple de 10 à 90 % en poids, et plus préférentiellement entre 30 et 70 %.

On donne ci-après, le schéma réactionnel du procédé de l'invention pour faciliter la compréhension de l'exposé de l'invention, sans pour autant lier la portée de l'invention, à celui-ci. 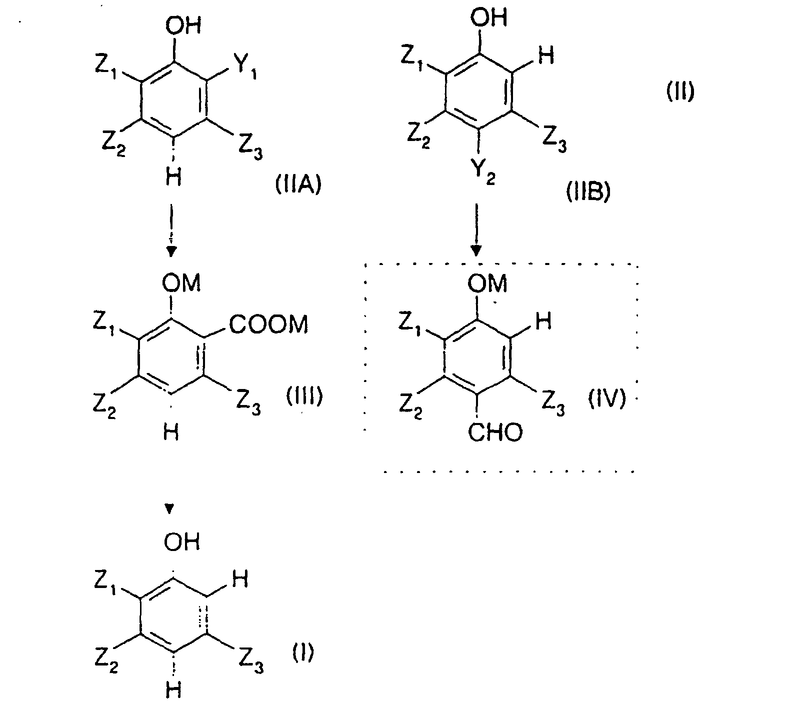 dans lesdites formules (I) à (IV) :
- Y₁ et Y₂, identiques ou différents, représentent l'un des groupes suivants :
   . un groupe - CHO,
   . un groupe - CH₂OH,
- M représente un atome d'hydrogène et/ou un cation métallique du groupe (la) ou (IIa) de la classification périodique ou un cation ammonium,
- Z₁, Z₂, Z₃, ont les significations données précédemment.

Dans le présent texte, on se réfère ci-après à la Classification périodique des éléments publiée dans le Bulletin de la Société Chimique de France, n°1 (1966).

Conformément au procédé de l'invention, on effectue une oxydation sélective du groupe Y₁ en position 2 en groupe carboxylique d'un composé phénolique (A) répondant préférentiellement à la formule (IIA) et éventuellement d'un groupe hydroxyméthyle en position 4 en groupe formyle d'un composé phénolique (B) répondant préférentiellement à la formule (IIB).

L'oxydation est réalisée par l'oxygène moléculaire ou un gaz en contenant, en présence généralement d'un catalyseur.

Un mode d'oxydation préférée consiste à oxyder un mélange de composés phénoliques de formule (II), en phase liquide, à l'aide d'oxygène moléculaire ou un gaz en contenant, en milieu aqueux renfermant un agent basique, en présence d'un catalyseur à base d'un métal M₁ choisi parmi les métaux du groupe 1b et 8 de la classification périodique des éléments comprenant éventuellement, à titre d'activateurs des métaux tels que le cadmium, le cérium, le bismuth, le plomb, l'argent, le tellure ou l'étain.

Conformément à l'invention, il a été trouvé d'une manière totalement surprenante que, si l'on augmentait la température et que l'on conduisait la réaction de préférence sous pression ou si l'on augmentait la quantité de la base présente lors de l'oxydation, on oxydait sélectivement le groupe formyle ou hydroxyméthyle en position 2 de la molécule (A) en groupe carboxylique, et le groupe situé en position 4 de la molécule (B) étant au plus oxydé en groupe formyle.

Les catalyseurs intervenant dans le procédé de l'invention sont à base d'un métal du groupe 1b et 8 de la classification périodique.

Comme exemples de catalyseurs à base d'un métal du groupe 8 de la classification périodique, on peut citer le nickel, le ruthénium, le rhodium, le pallàdium, l'osmium, l'iridium, le platine et leurs mélanges. Pour ce qui est des métaux du groupe 1b, on préfère faire appel au cuivre.

On utilise de préférence, des catalyseurs de platine et/ou de palladium, pris sous toutes les formes disponibles telles que par exemple : le noir de platine, le noir de palladium, l'oxyde de platine, l'oxyde de palladium ou le metal noble lui-même déposé sur des supports divers tels que le noir de carbone, le carbonate de calcium, les alumines et silices activées ou des matériaux équivalents. Des masses catalytiques à base de noir de carbone conviennent particulièrement.

La quantité de ce catalyseur à mettre en oeuvre, exprimée en poids de métal M₁ par rapport à celui du mélange de composés phénoliques de formule (II) peut varier de 0,01 à 10 % et, de préférence, de 0,04 à 2 %.

Pour plus de détails sur les catalyseurs, on peut se référer à US-A-3 673 257, FR-A-2 305 420, FR-A-2 350 323.

Dans les catalyseurs intervenant dans le procédé de l'invention, on met en oeuvre préférentiellement un activateur.

L'activateur peut être choisi parmi tous ceux mentionnés dans les brevets précités. De préférence, on fait appel au bismuth, au plomb et au cadmium, sous forme de métaux libres ou de cations. Dans ce dernier cas, l'anion associé n'est pas critique et on peut utiliser tous dérivés de ces métaux. De préférence, on met en oeuvre le bismuth métal ou ses dérivés.

On peut faire appel à un dérivé minéral ou organique du bismuth dans lequel l'atome de bismuth se trouve à un degré d'oxydation supérieur à zéro, par exemple égal à 2, 3, 4 ou 5. Le reste associé au bismuth n'est pas critique dès l'instant qu'il satisfait à cette condition. L'activateur peut être soluble ou insoluble dans le milieu réactionnel.

Des composés illustratifs d'activateurs qui peuvent être utilisés dans le procédé selon la présente invention sont : les oxydes de bismuth ; les hydroxydes de bismuth ; les sels d'hydracides minéraux tels que : chlorure, bromure, iodure, sulfure, séléniure, tellure de bismuth : les sels d'oxyacides minéraux tels que : sulfite, sulfate, nitrite, nitrate, phosphite, phosphate, pyrophosphate, carbonate, perchlorate, antimoniate, arséniate, sélénite, séléniate de bismuth ; les sels d'oxyacides dérivés de métaux de transition tels que : vanadate, niobate, tantalate, chromate, molybdate, tungstate, permanganate de bismuth.

D'autres composés appropriés sont également des sels d'acides organiques aliphatiques ou aromatiques tels que : acétate, propionate, benzoate, salicylate, oxalate, tartrate, lactate, citrate de bismuth ; des phénates tels que : gallate et pyrogallate de bismuth. Ces sels et phénates peuvent être aussi des sels de bismuthyle.

Comme autres composés minéraux ou organiques, on peut utiliser des combinaisons binaires du bismuth avec des éléments tels que phosphore et arsenic ; des hétéropplyacides contenant du bismuth ainsi que leurs sels ; conviennent également les bismuthines aliphatiques et aromatiques.

A titre d'exemples spécifiques, on peut citer:
- comme oxydes : BiO ; Bi₂O₃ : Bi₂O₄; Bi₂O_{5.}
- comme hydroxydes : Bi(OH)₃,
- comme sels d'hydracides minéraux : le chlorure de bismuth BiCl₃ ; le bromure de bismuth BiBr₃ ; l'iodure de bismuth Bil₃ ; le sulfure de bismuth Bi₂S₃ ; le séléniure de bismuth Bi₂Se₃ : le tellure de bismuth Bi₂Te₃,
- comme sels d'oxyacides minéraux ; le sulfite basique de bismuth Bi₂(SO₃)₃, Bi₂O₃, 5H₂O ; le sulfate neutre de bismuth Bi₂(SO₄)₃ ; le sulfate de bismuthyle (BiO)HSO₄ ; le nitrite de bismuthyle (BiO)NO₂, 0,5H₂O ; le nitrate neutre de bismuth Bi(NO₃)₃, 5H₂O ; le nitrate double de bismuth et de magnésium 2Bi(N0₃)₃, 3Mg(NO₃)₂, 24H₂O ; le nitrate de bismuthyle (BiO)NO₃ ; le phosphite de bismuth Bi₂(PO₃H)₃, 3H₂O ; le phosphate neutre de bismuth BiPO₄ ; le pyrophosphate de bismuth Bi₄(P₂O₇)₃ ; le carbonate de bismuthyle (BiO)₂CO₃, O,5H₂O ; le perchlorate neutre de bismuth Bi(ClO₄)₃, 5H₂O ; le perchlorate de bismuthyle (BiO)ClO₄ ; l'antimoniate de bismuth BiSbO₄ ; l'arséniate neutre de bismuth Bi(AsO₄)₃ ; l'arséniate de bismuthyle (BiO)AsO₄, 5H₂O ; le sélénite de bismuth Bi₂(SeO₃)₃.
- comme sels d'oxyacides dérivés de métaux de transition : le vanadate de bismuth BiVO₄ ; le niobate de bismuth BiNbO₄ : le tantalate de bismuth BiTaO₄ ; le chromate neutre de bismuth Bi₂(CrO₄) ; le dichromate de bismuthyle [(BiO)₂]₂Cr₂O₇ ; le chromate acide de bismuthyle H(BiO)CrO₄ ; le chromate double de bismuthyle et de potassium K(BiO)CrO₄ ; le molybdate de bismuth Bi₂(MoO₄)₃ ; le tungstate de bismuth Bi₂(WO₄)₃ ; le molybdate double de bismuth et de sodium NaBi(MoO₄)₂ ; le permanganate basique de bismuth Bi₂O₂(OH)MnO₄.
- comme sels d'acides organiques aliphatiques ou aromatiques : l'acétate de bismuth Bi(C₂H₃O₂)₃ ; le propionate de bismuthyle (BiO)C₃H₅O₂ ; le benzoate basique de bismuth C₆H₅CO₂Bi(OH)₂ ; le salicylate de bismuthyle C₆H₄CO₂(BiO)(OH) ; l'oxalate de bismuth (C₂O₄)₃Bi₂ ; le tartrate de bismuth Bi₂(C₄H₄O₆)₃, 6H₂O ; le lactate de bismuth (C₆H₉O₅)OBi, 7H₂O ; le citrate de bismuth C₆H₅O₇Bi.
- comme phénates : le gallate basique de bismuth C₇H₇O₇Bi ; le pyrogallate basique de bismuth C₆H₃(OH)₂(OBi)(OH).

Comme autres composés minéraux ou organiques conviennent également : le phosphure de bismuth BiP ; l'arséniure de bismuth Bi₃As₄ ; le bismuthate de sodium NaBiO₃ ; les acides bismuth-thiocyaniques H₂[Bi(BNS)₅], H₃[Bi(CNS)₆] et leurs sels de sodium et potassium ; la triméthylbismuthine Bi(CH₃)₃, la- triphénylbismuthine Bi(C₆H₅)₃.

Les dérivés du bismuth qui sont utilisés de préférence pour conduire le procédé selon l'invention sont : les oxydes de bismuth ; les hydroxydes de bismuth ; les sels de bismuth ou de bismuthyle d'hydracides minéraux ; les sels de bismuth ou de bismuthyle d'oxyacides minéraux ; les sels de bismuth ou de bismuthyle d'acides organiques aliphatiques ou aromatiques ; et les phénates de bismuth ou de bismuthyle.

Un groupe d'activateurs qui conviennent particulièrement bien à la réalisation de l'invention est constitué par : les oxydes de bismuth Bi₂O₃ et Bi₂O₄; l'hydroxyde de bismuth Bi(OH)₃ ; le sulfate neutre de bismuth Bi₂(SO₄)₃ ; le chlorure de bismuth BiCl₃ ; le bromure de bismuth BiBr₃ ; l'iodure de bismuth Bil₃ ; le nitrate neutre de bismuth Bi(NO₃)₃, 5H₂O ; le nitrate de bismuthyle BiO(NO₃) ; le carbonate de bismuthyle (BiO)₂CO₃, O,5H₂O ; l'acétate de bismuth Bi(C₂H₃O₂)₃ ; le salicylate de bismuthyle C₆H₄CO₂(BiO)(OH).

La quantité d'activateur utilisée, exprimée par la quantité de métal contenue dans l'activateur par rapport au poids du metal M₁ engagé, peut varier dans de larges limites. Par exemple, cette quantité peut être aussi petite que 0,1 % et peut atteindre le poids de métal M₁ engagé et même le dépasser sans inconvénient.

Plus particulièrement, cette quantité est choisie de manière qu'elle apporte dans le milieu d'oxydation de 10 à 900 ppm en poids de métal activateur par rapport au mélange de composés phénoliques de formule (II). A cet égard, des quantités supérieures d'activateur de l'ordre de 900 à 1500 ppm peuvent naturellement être utilisées, mais sans avantage supplémentaire important.

Selon le procédé de l'invention, l'oxydation est conduite dans un milieu aqueux contenant en solution un agent basique, et plus particulièrement l'hydroxyde d'ammonium, les bases alcalines ou alcalino-terreuses parmi lesquelles on peut citer des hydroxydes tels que l'hydroxyde de sodium, de potassium, de lithium et la baryte ; des alcanolates alcalins tels que le méthylate, l'éthylate, l'isopropylate et le tert-butylate de sodium ou de potassium, des carbonates ou bicarbonates de sodium ou de potassium et de façon générale, les sels des bases alcalines ou alcalino-terreuses et d'acides faibles.

Ainsi, les composés de formule (III) et (IV) peuvent être totalement ou partieilement salifiés selon la quantité d'agent basique mise en oeuvre. Il s'ensuit que dans lesdites formules, M symbolise un atome d'hydrogène et/ou un cation métallique du groupe (Ia) ou (IIa) ou un cation ammonium.

Pour des considérations économiques, on fait appel à l'hydroxyde de sodium ou de potassium. La proportion de base minérale à utiliser peut être comprise entre 0,5 et 10 moles, de préférence, entre 1 et 4 moles, et encore plus préférentiellement entre 2 et 4 moles de base minérale par mole de composés phénoliques de formule (II).

La concentration pondérale du mélange de composés phénoliques de formule (II) dans la phase liquide est habituellement comprise entre 1 % et 60 %, de préférence entre 2 % et 30 %.

Pratiquement une manière d'exécuter le procédé consiste à mettre en contact avec de l'oxygène moléculaire ou un gaz en contenant, par exemple l'air, la solution renfermant le mélange de composés phénoliques de formule (II), l'agent basique, le catalyseur à base de métal M₁, éventuellement l'activateur, selon les proportions indiquées ci-dessus.

On peut opérer à pression atmosphérique, mais l'on préfère travailler sous pression entre 1 et 20 bar.

Le mélange est ensuite agité à la température désirée jusqu'à consommation d'une quantité d'oxygène correspondant à celle nécessaire pour transformer le groupe hydroxyméthyle ou formyle du composé (A) en groupe carboxylique et éventuellement le groupe hydroxyméthyle du composé (B) en groupe formyle.

La température réactionnelle à adopter varie selon la stabilité thermique des produits à préparer.

Conformément à l'invention, la température est choisie de préférence, dans une gamme de température allant de 30°C à 200°C, de préférence, entre 40°C et 160°C.

La température est à adapter par l'Homme du Métier en fonction des conditions réactionnelles (en particulier quantité de base, nature du métal M₁, pression et agitation). Il a été trouvé notamment que plus la température est basse, plus la quantité d'agent basique à mettre en oeuvre est choisie élevée.

On précisera, à titre d'exemples, les conditions préférées dans le cas des métaux préférés, platine et palladium. Pour le platine, la température étant choisie entre 100°C à 160 °C, la quantité de base à utiliser est avantageusement comprise entre 1 et 3 moles, par mole de composés phénoliques de formule (II). Dans le cas du palladium. la température peut être choisie entre 30°C et 200°C, de préférence, entre 30°C et 150°C et pour ce dernier intervalle, la quantité de base est préférentiellement de 2 à 4 moles par mole de composés phénoliques.

Ainsi, la quantité de base doit être suffisante pour oxyder le groupement Y₁ en position ortho en groupe carboxylique. Elle est déterminée par l'Homme du Métier, en fonction de la température et du métal choisi.

En fin de réaction qui dure de préférence, entre 30 minutes et 6 heures, on récupère l'acide 2-hydroxybenzoique qui peut être partiellement ou totalement salifié et répondant préférentiellement à la formule (III) et un 4-hydroxybenzaldéhyde répondant préférentiellement à la formule (IV).

Puis, après refroidissement s'il y a lieu, on sépare la masse catalytique du milieu réactionnel, par exemple par filtration.

A partir du milieu réactionnel, on va récupérer le 4-hydroxybenzaldéhyde.

Un premier mode de traitement du milieu réactionnel consiste à effectuer une extraction du 4-hydroxybenzaldéhyde à pH contrôlé.

A cet effet, on met en contact le milieu réactionnel avec un solvant organique susceptible d'extraire l'aldéhyde.

Dans le choix du solvant, on choisit un solvant non miscible à l'eau.

Comme exemples de solvants convenant à l'invention, on peut citer notamment les cétones telles que la méthyléthylcétone, la méthylisobutylcétone, la cyclohexanone ; les esters tels que l'acétate d'éthyle, l'acétate d'isopropyle, l'acétate de butyle ; les éther-oxydes tels que le diéthyléther, le diisopropyléther, le méthyl-tert-butyléther, l'éthyl-tert-butyléther, le di-n-butyléther ; les alcools lourds (ayant de préférence au moins 4 atomes de carbone) tels que le butanol, l'hexanol, l'octanol, le cyclohexanol ; les hydrocarbures aliphatiques tels que le n-pentane, l'hexane, l'heptane et le cyclohexane ; les hydrocarbures aliphatiques halogénés tels que le dichlorométhane, le dichloroéthane ; les hydrocarbures aromatiques tels que le toluène, les xylènes ; les hydrocarbures aromatiques halogénés tels que le monochlorobenzène, le dichlorobenzène et leurs mélanges.

On met en contact ainsi le milieu réactionnel avec le solvant réactionnel, généralement un volume de solvant par volume de milieu.

On peut effectuer de 1 à plusieurs extractions, par exemple 5, et plus préférentiellement de 1 à 3.

Avant l'addition du solvant ou simultanément, on ramène le pH entre 4 et 9, par addition d'un acide protonique d'origine minérale, de préférence l'acide chlorhydrique ou l'acide sulfurique ou d'un acide organique tel que par exemple, l'acide triflurométhanesulfonique ou l'acide méthanesulfonique. La concentration de l'acide est indifférente et l'on fait appel de préférence, aux formes commerciales.

On sépare les phases aqueuse et organique.

La phase aqueuse comprend l'acide 2-hydroxybenzoïque sous forme salifiée.

La phase organique comprend le 4-hydroxybenzaldéhyde qui est peut être alors récupéré selon les techniques classiques, notamment par distillation.

On peut traiter la phase aqueuse en effectuant une décarboxylation dont la description est détaillée dans la deuxième variante, de l'acide 2-hydroxybenzoïque obtenu ce qui permet de régénérer le composé phénolique de départ qui peut être alors recyclé.

Selon une deuxième variante du procédé de l'invention, en fin de réaction conduisant à l'acide 2-hydroxybenzoïque partiellement ou totalement salifié et au 4-hydroxybenzaldéhyde, on soumet le milieu réactionnel à une réaction de décarboxylation.

Pour ce faire, on acidifie le milieu résultant par addition d'un acide protonique d'origine minérale, notamment ceux cités précédemment jusqu'à obtention d'un pH inférieur ou égal à 3.

On chauffe le milieu réactionnel à une température variant par exemple entre 120°C et 350°C et, de préférence, de 150°C et 220°C.

Le procédé est conduit, de préférence, sous la pression autogène des réactifs.

En fin de réaction, on refroidit le milieu réactionnel entre 20°C et 80°C.

On obtient un milieu bi-phasique constitué d'une phase organique comprenant d'une part, le 4-hydroxybenzaldéhyde répondant préférentiellement à la formule (IV) et le composé phénolique de départ de formule (I) et d'autre part une phase aqueuse saline.

On sépare les phases organique et aqueuse et l'on récupère le 4-hydroxybenzaldéhyde à partir de la phase organique, selon les techniques classiques de séparation, par exemple par extraction à l'aide d'un solvant approprié et ensuite par distillation. On peut se référer à la description de la première variante.

Conformément au procédé de l'invention, on part d'un mélange de deux composés phénoliques, l'un portant un groupe formyle ou hydroxyméthyle en position 2 et l'autre portant un groupe formyle ou hydroxyméthyle en position 4.

Ainsi, les mélanges de départ (IIA) et (IIB) répondent plus particulièrement aux formules données ci-après : 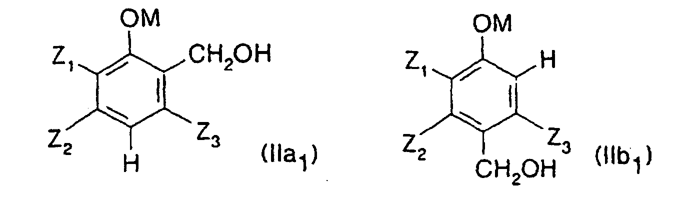 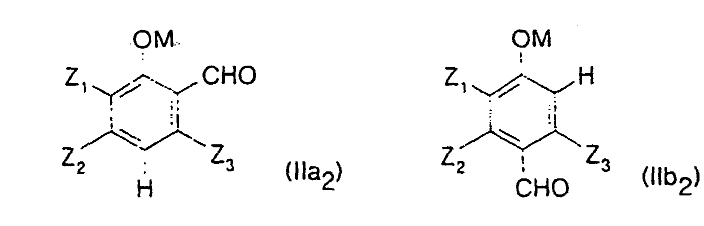 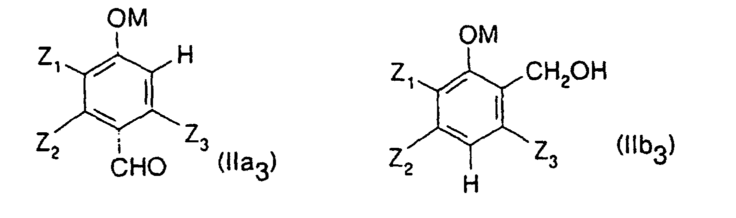 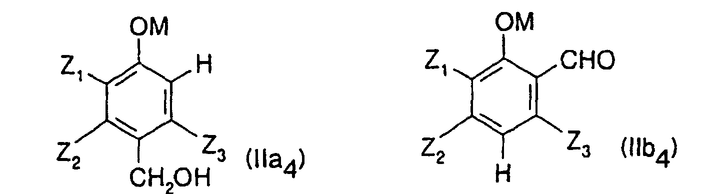 dans lesdites formules, M, Z₁, Z₂, Z₃, ont les significations données précédemment.

Les mélanges de composés phénoliques, auxquels on peut appliquer le procédé selon l'invention sont des produits généralement connus qui peuvent être préparés par diverses méthodes de synthèse organique.

Ainsi, des mélanges de formule (IIa₁) et (IIb₁) peuvent être obtenus par un procédé d'hydroxyméthylation d'un phénol par condensation de celui-ci avec du formaldéhyde ou d'un composé générateur de formaldéhyde en phase aqueuse en présence d'une base alcaline ou alcalino-terreuse.

Plus précisément, on part d'un phénol non substitué sur les positions ortho et para par rapport au groupe hydroxyle, de formule générale (I) : 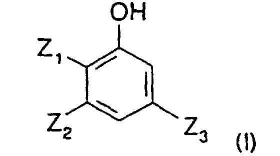 dans laquelle Z₁, Z₂, Z₃, ont les significations données précédemment.

Parmi les phénols de formule (I) qui peuvent servir de point de départ à la synthèse des composés de formule (II), on peut citer le phénol, la pyrocatéchine, le gaïacol, le guétol, le 3-méthoxyphénol, le 3-éthoxyphénol, le 3-isopropoxyphénol, le 3-t-butoxyphénol, le m-crésol, l'o-crésol.

Les conditions choisies pour le déroulement de cette étape d'hydroxyméthylation sont celles enseignées par l'art antérieur rappelé ci-après : cf. notamment H.G. PEER Rec. Trav. Chim. Pays-Bas 79 825-835 (1960) ; GB-A-774 696 ; GB-A-751 845 ; EP-A-165 ; J.H. FREEMAN J. Am. Chem. Soc. 74 6 257-6 260 (1952) et 76 2080-2087 (1954) ; H.G. PEER Rec. Trav. Chim. Pays-Bas 78 851-863 (1959) ; H. EULER et al Arkiv für Chem. 13 1-7 (1939) ; P. CLAUS et al Monath. Chem. 103 1178-11293 (1972).

On peut faire appel au formaldéhyde ou tout générateur de formaldéhyde tel que, par exemple, le trioxane ou le paraformaldéhyde utilisé sous la forme de polyformaldéhydes linéaires de degré de polymérisation indifférent, ayant de préférence un nombre de motifs (CH₂O) compris ente 8 et 100 motifs,

Le formaldéhyde peut être utilisé, sous forme de solution aqueuse dont la concentration n'est pas critique. Elle peut varier entre 20 et 50 % en poids : on utilise de préférence les solutions commerciales dont la concentration est d'environ 30 à 40 % en poids.

La quantité de formaldéhyde exprimée en moles de formaldéhyde par mole de phénol peut varier dans de larges limites. Le rapport molaire formaldéhyde/phénol peut varier entre 0,5 et 2,0 et de préférence entre 0,5 et 1,5.

La quantité de base présente dans le milieu d'hydroxyméthylation exprimée par le nombre de moles de base/hydroxyle phénolique du phénol à hydroxyméthyler peut varier dans de larges limites. En général ce rapport, variable suivant la nature de la base, peut varier entre 0,1 et 2 et, de préférence, entre 0,5 et 1,1. Comme base, on peut utiliser celles citées plus haut pour la phase d'oxydation. L'emploi des hydroxydes alcalins en solution aqueuse est particulièrement commode.

En général, l'étape d'hydroxyméthylation est conduite à une température comprise entre 0 et 100°C et de préférence entre 20 et 70°C.

Le procédé est conduit, de préférence, sous la pression autogène des réactifs pour éviter les pertes éventuelles du paraformaldéhyde qui peut être gazeux aux températures de mises en oeuvre.

On préfère conduire la réaction sous atmosphère contrôlée de gaz inertes tels que l'azote ou les gaz rares, par exemple l'argon.

La durée de la réaction peut être très variable. Elle se situe, le plus souvent, entre 30 minutes et 24 heures, de préférence entre 4 heures et 8 heures.

D'un point de vue pratique, la réaction est aisément réalisée en chargeant dans l'appareillage le phénol et le formaldéhyde, éventuellement une base, puis en portant le mélange réactionnel sous agitation à la température désirée, pendant la durée nécessaire à l'achèvement de la réaction.

L'ordre d'introduction des réactifs n'est pas critique et peut donc être différent.

On obtient un mélange de composés phénoliques de formule (IIa₁) et (IIb₁).

Les composés de formule (IIa₂) et (IIb₂) peuvent être préparés par oxydation des composés phénoliques hydroxyméthylés de formule (IIa₁) et (IIb₁), par oxydation par l'oxygène moléculaire ou un gaz qui en contient, en phase aqueuse alcaline en présence d'un catalyseur à base d'un métal du groupe 8 de la classification périodique, de préférence, le platine et le palladium, contenant éventuellement à titre d'activateur des métaux tels que le cadmium, le cérium, le bismuth, le plomb, l'argent, le tellure ou l'étain. De tels procédés ont été décrits dans US-A-3 673 257, FR-A-2 305 420 et dans FR-A-2 350 323.

Si cela est nécessaire, le pH de la solution est porté à une valeur comprise entre 8 et 13 par addition éventuelle d'une base alcaline ou alcalino-terreuse. La valeur optimale du pH dépend de la nature des phénols hydroxyméthylés.

Par exemple, dans le cas d'un catalyseur au platine, la quantité de base à mettre en oeuvre est avantageusement comprise entre 1 et 3 moles, par mole de composés phénoliques hydroxyméthylés et entre 0,5 et 2 dans le cas d'un catalyseur au palladium.

La température de la réaction d'oxydation se situe entre 10°C et 60°C, et de préférence entre 20°C et 50°C.

Plus spécifiquement encore le procédé selon la présente invention convient bien à la préparation de composés de formule (IIa₂) et (IIb₂) à partir de composés phénoliques de formule (IIa₁) et (IIb₁) résultant de la première étape, par l'oxygène moléculaire ou un gaz qui en contient, en présence d'un catalyseur à base d'un métal du groupe 8 de la classification périodique, contenant éventuellement un métal tel que ceux utilisés à titre d'activateur, sans séparation intermédiaire des composés phénoliques hydroxyméthylés.

Il apparaît particulièrement avantageux du point de vue industriel pour la mise en oeuvre du procédé selon la présente invention de faire appel à des composés de formule (IIa₂) et (IIb₂) obtenus par un procédé en deux étapes comprenant :
- l'hydroxyméthylation d'un phénol en milieu aqueux en présence d'une base alcaline ou alcalino-terreuse par le formaldéhyde ou un composé générateur de formaldéhyde, conduisant à un mélange de composés phénoliques hydroxyméthylés, l'un hydroxyméthylé en position 2 et l'autre en position 4,
- et l'oxydation, sans séparation intermédiaire, des composés phénoliques obtenus par l'oxygène moléculaire ou un gaz qui en contient, en phase aqueuse alcaline en présence d'un catalyseur à base d'un métal du groupe 8 de la classification périodique, éventuellement à titre d'activateur, un métal tel que ceux cités précédemment.

Un intérêt supplémentaire du procédé de l'invention est qu'il permet de mettre en oeuvre des mélanges de composés phénoliques issus directement des étapes précédentes d'hydroxyméthylation éventuellement d'oxydation.

Comme mentionné précédemment, le procédé de l'invention est particulièrement bien adapté pour ia préparation de la vanilline et de l'ethylvanilline à partir d'un mélange de composés phénolique obtenus par hydroxyméthylation du gaïacol ou du guétol.

Ainsi, la vanilline peut être préparée en soumettant un mélange de composés phénoliques, o-hydroxyméthylgaïacol (A) et p-hydroxyméthylgaïacol (B) à une oxydation sélective du groupe hydroxyméthyle en position 2 du composé (A) en groupe carboxylique, et du groupe hydroxyméthyle du composé (B) en position 4 en groupe formyle, conduisant ainsi à un mélange d'un acide 2-hydroxy-3-méthoxybenzoïque et de vanilline puis à récupérer cette dernière.

Une autre variante consiste à soumettre un mélange de composés phénoliques, o-formylgaïacol (A) et p-formylgaïacol (B) à une oxydation sélective du groupe formyle en position 2 du composé (A) en groupe carboxylique, conduisant ainsi à un mélange d'un acide 2-hydroxy-3-méthoxybenzoïque et de vanilline puis à récupérer cette dernière.

En ce qui concerne la préparation d'éthylvanilline, selon l'invention, on soumet un mélange de composés phénoliques, o-hydroxyméthylguétol (A) et p-hydroxyméthylguétol (B) à une oxydation sélective du groupe hydroxyméthyle en position 2 du composé (A) en groupe carboxylique, et du groupe hydroxyméthyle du composé (B) en position 4 en groupe formyle, conduisant ainsi à un mélange d'un acide 2-hydroxy-3-éthoxybenzoïque et d'éthylvanilline puis à récupérer cette dernière.

Une autre variante réside dans le fait que l'on part d'un mélange de composés phénoliques, o-formylguétol (A) et p-formylguétol (B) à une oxydation sélective du groupe formyle en position 2 du composé (A) en groupe carboxylique, conduisant ainsi à un mélange d'un acide 2-hydroxy-3-éthoxybenzoïque et d'éthylvanilline puis à récupérer cette dernière.

On donne ci-après des exemples de réalisation de l'invention. Ces exemples sont donnés à titre illustratif et sans caractère limitatif.

Dans les exemples, on définit le taux de conversion et le rendement obtenu.

Le taux de conversion (TT) correspond au rapport entre le nombre de moles de substrat transformées et le nombre de moles de substrat engagées.

Le rendement (RR) correspond au rapport entre le nombre de moles de produit formées et le nombre de moles de substrat engagées.

Le rendement (RTᵥₐₙᵢₗₗᵢₙₑ) correspond au rapport entre le nombre de moles de vanilline formées et le nombre de moles de gaïacol transformées sur l'enchaînement.

Dans les exemples, les abréviations signifient :
- o-hydroxyméthylgaïacol = OMG
- p-hydroxyméthylgaïacol = PMG
- o-vanilline = 3-méthoxy-2-hydroxybenzaldéhyde = OVA
- p-vanilline = 3-méthoxy-4-hydroxybenzaldéhyde = PVA
- acide o-vanillique = acide 2-hydroxy-3-méthoxybenzoïque = AOV
- acide p-vanillique = acide 4-hydroxy-3-méthoxybenzoïque = APV

### EXEMPLES

### Exemple 1:

Dans cet exemple, on effectue l'oxydation d'un mélange d'ohydroxyméthylgaïacol et de p-hydroxyméthylgaïacol.

Dans un autoclave sous pression de 3,9 l, muni d'une turbine auto-aspirante, on introduit 2700 g d'une solution aqueuse contenant 28,5 g d'o-hydroxyméthylgaïacol (OMG) et 33,72 g de p-hydroxyméthylgaïacol (PMG) et 148 g de soude.

Cette solution aqueuse est issue d'une condensation du gaïacol sur le formol en milieu aqueux basique, et préparée comme décrit dans l'état de la technique (notamment selon l'exemple 4 de US 4 351 962).

On ajoute à ce milieu réactionnel 0,54 g de trioxide de bismuth et 22 g de catalyseur au palladium déposé sur noir à raison de 3 % en poids de métal.

On met sous agitation à 1500 t/min, on monte la température du milieu réactionnel à 45° C sous azote.

On se place alors sous une pression de 3 bar et l'on introduit dans le milieu réactionnel de l'air avec un débit de 300 g/l.

On maintient le milieu réactionnel 6 h dans ces conditions.

On refroidit le mélange réactionnel et l'on ramène la pression à la pression atmosphérique, puis l'on filtre le catalyseur.

Le milieu réactionnel est alors dosé par chromatographie liquide haute performance.

Les résultats obtenus sont les suivants :

| | |
|---|---|
| TT OMG = | 100% |
| RR o-vanilline = | 7% |
| RR o-vanillique = | 93 % |

| | |
|---|---|
| TT PMG = | 100 % |
| RR p-vanilline = | 89 % |
| RR acide p-vanillique = | 7 % |

Il ressort de l'examen de ces resultats que l'o-vanilline a été sélectivement oxydée comparativement à la p-vanilline.

## Revendications

1. Procédé de préparation d'un acide 2-hydroxybenzoïque et d'un 4-hydroxybenzaldéhyde et dérivés **caractérisé par le fait que** l'on soumet un mélange de composés phénoliques, l'un (A) porteur d'un groupe formyle ou hydroxyméthyle en position 2, et l'autre (B) porteur d'un groupe formyle ou hydroxyméthyle en position 4, à une oxydation sélective du groupe formyle ou hydroxyméthyle en position 2 du composé (A) en groupe carboxylique, et éventuellement d'un groupe hydroxyméthyle du composé (B) en position 4 en groupe formyle, conduisant ainsi à un mélange d'un acide 2-hydroxybenzoïque et d'un 4-hydroxybenzaldéhyde, en présence d'un catalyseur à base de palladium et/ou de platine et une base mise en oeuvre en une quantité telle que le rapport entre le nombre de moles d'agent basique et le nombre de moles de composés phénoliques est compris entre 2 et 10.

2. Procédé selon la revendication 1 **caractérisé par le fait que** le mélange de composés phénoliques mis en oeuvre répond à la formule Générale (II) : 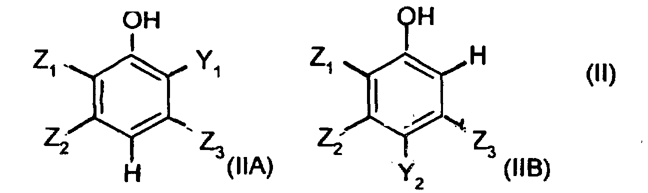 dans lesdites formules (IIA) et (IIB):
- Y₁ et Y₂, identiques ou différents, représentent l'un des groupes suivants :
. un groupe - CHO,
. un groupe -CH₂OH,
- Z₁, Z₂ et Z₃, identiques ou différents, représentent un atome d'hydrogène, un radical alkyle, alcényle, alkoxy, hydroxyalkyle, alkoxyalkyle, cycloalkyle, aryle, un groupe hydroxyle, un groupe nitro, un atome d'halogène, un groupe trifluorométhyle.

3. Procédé selon l'une des revendications 1 et 2 **caractérisé par le fait que** le composés phénoliques répondent à la formule (IIA) et (IIB) dans lesquelles Z₁, Z₂ et Z₃, identiques ou différents, représentent l'un des atomes ou groupes suivants :
. un atome d'hydrogène,
. un radical alkyle, linéaire ou ramifié, ayant de 1 à 12 atomes de carbone, de préférence de 1 à 4 atomes de carbone, de préférence méthyle, éthyle, propyle, isopropyle, butyle, isobutyle, sec-butyle, tert-butyle,
. un radical alcényle, linéaire ou ramifié, ayant de 2 à 12 atomes de carbone, de préférence de 2 à 4 atomes de carbone, tde préférence vinyle, allyle,
. un radical alkoxy linéaire ou ramifié ayant de 1 à 12 atomes de carbone, de préférence de 1 à 4 atomes de carbone de préférence les radicaux méthoxy, éthoxy, propoxy, isopropoxy, butoxy, isobutoxy, sec-butoxy, tert-butoxy,
. un radical phényle,
. un atome d'halogène, de préférence un atome de fluor, chlore ou brome.

4. Procédé selon l'une des revendications 1 à 3 **caractérisé par le fait que** les composés phénoliques répondent aux formules (IIA) et (IIB) dans lesquelles Z₁ représente un atome d'hydrogène ou un radical alkyle ou alkoxy, linéaire ou ramifié, ayant de 1 à 6 atomes de carbone, de préférence, 1 à 4 atomes de carbone ; Z₂ et Z₃ représentent un atome d'hydrogène ; Y₁ et Y₂, identiques, représentent un groupe formyle ou un groupe hydroxyméthyle.

5. Procédé selon l'une des revendications 1 à 4 **caractérisé par le fait que** le mélange de composés phénoliques répondant à la formule (II) est :
- l'o-hydroxyméthylphénol et le p-hydroxyméthylphénol,
- l'o-hydroxyméthylgaïacol et le p-hydroxyméthylgaïacol,
- l'o-formylgaïacol et le p-formylgaïacol.
- l'o-hydroxyméthylguétol et le p-hydroxyméthylguétol,
- l'o-formylguétol et le p-formylguétol.

6. Procédé selon l'une des revendications 1 à 5 **caractérisé par le fait que** l'on oxyde le mélange de composés phénoliques de formule (II), en phase liquide, à l'aide d'oxygène moléculaire ou un gaz en contenant, en milieu aqueux renfermant un agent basique, en présence d'un catalyseur à base de platine et/ou de palladium comprenant éventuellement, à titre d'activateurs des métaux choisis parmi le cadmium; le cérium, le bismuth, le plomb, l'argent, le tellure ou l'étain.

7. Procédé selon la revendication 6 **caractérisé par le fait que** le catalyseur au platine et/ou palladium, est apporté sous forme de noir de platine, de noir de palladium, d'oxyde de platine, d'oxyde de palladium ou de métal noble lui-même déposé sur des supports divers tels que le nojr de carbone, le carbonate de calcium, les alumines et silices activées ou des matériaux équivalents, de préférence le noir de carbone.

8. Procédé selon l'une des revendications 6 et 7 **caractérisé par le fait que** la quantité de catalyseur à mettre en oeuvre, exprimée en poids de métal par rapport à celui du mélange de composés phénoliques de formule (II) peut varier de 0,01 à 10 % et, de préférence, de 0,04 à 2 %.

9. Procédé selon l'une des revendication 6 à 8 **caractérisé par le fait que** l'activateur est un dérivé organique ou inorganique du bismuth pris dans le groupe formé par : les oxydes de bismuth ; les hydroxydes de bismuth ; les sels de bismuth ou de bismuthyle d'hydracides minéraux, de préférence les chlorure, bromure, iodure, sulfure, sélénure, tellure ; les sels de bismuth ou de bismuthyle d'oxyacides minéraux, de préférence les sulfite, sulfate, nitrite, nitrate, phosphite, phosphate, pyrophosphate, carbonate, perchlorate, antimoniate, arséniate, sélénite, séléniate ; les sels de bismuth ou de bismuthyle d'acides organiques aliphatiques ou aromatiques, de préférence les acétate, propionate, salicylate, benzoate, oxalate, tartrate, lactate, citrate ; les phénates de bismuth ou de bismuthyle, de préférence les gallate et pyrogallate.

10. Procédé selon la revendication 9 **caractérisé par le fait que** le dérivé du bismuth est pris dans le groupe formé par : les oxydes de bismuth Bi₂O₃ et Bi₂O₄ ; l'hydroxyde de bismuth Bi(OH)₃ ; le chlorure de bismuth BiCl₃ ; le bromure de bismuth BiBr₃ ; l'iodure de bismuth Bil₃ ; le sulfate neutre de bismuth Bi₂(SO₄)₃ ; le nitrate neutre de bismuth Bi(NO₃)₃, 5H₂O ; le nitrate de bismuthyle BiO(NO₃) ; le carbonate de bismuthyle (BiO)₂CO₃, 0,5 H₂O ; l'acétate de bismuth Bi(C₂H₃O₂)₃ ; le salicylate de bismuthyle C₆H₄CO₂(BiO)OH.

11. Procédé selon l'une des revendications 6 à 10 **caractérisé par le fait que** la quantité d'activateur est choisie de manière qu'elle apporte dans la milieu : d'une part, au moins 0,1 % en poids de métal activateur par rapport au poids du métal M₁ engagé, et d'autre part de 10 à 900 ppm en poids d'activateur par rapport au mélange de composés phénoliques de formule (II).

12. Procédé selon l'une des revendications 6 à 11 **caractérisé par le fait que** la réaction d'oxydation est conduite dans une gamme de température allant de 30°C à 200°C, de préférence, entre 40°C et 160°C.

13. Procédé selon l'une des revendications 6 à 12 **caractérisé par le fait que** l'on opère sous pression entre 1 et 20 bar.

14. Procédé selon l'une des revendications 1 à 13 **caractérisé par le fait que** l'oxydation est conduite dans un milieu aqueux contenant en solution un agent basique, de préférence, un hydroxyde de sodium, de potassium, en une quantité telle qu'elle représente de 2 à 10, de préférence de 2 à 4 moles de base minérale par mole de composés phénoliques de formule (II).

15. Procédé selon l'une des revendications 1 à 14 **caractérisé par le fait que** la température est choisie entre 30°C et 200°C, de préférence, entre 40°C et 160°C.

16. Procédé selon la revendication 15 **caractérisé par le fait que** dans le cas d'un catalyseur au platine, la température est choisie entre 100°C à 160 °C.

17. Procédé selon la revendication 15 **caractérisé par le fait que** dans le cas d'un catalyseur au palladium, la température est choisie entre 30°C et 200°C, de préférence, entre 30°C et 150°C ; la quantité de base à utiliser étant compris entre 2 et 4 moles, par mole de composés phénoliques de formule (II).

18. Procédé selon l'une des revendications 1 à 17 **caractérisé par le fait que** les substrats de départ répondent aux formules : 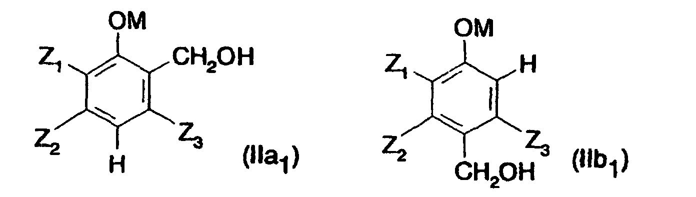 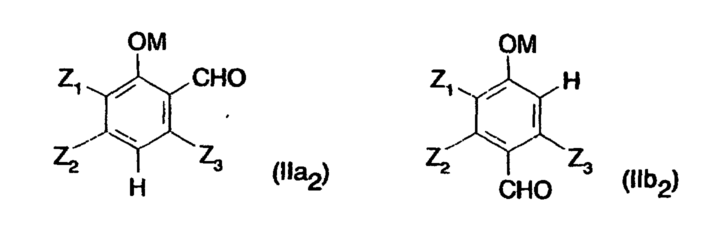 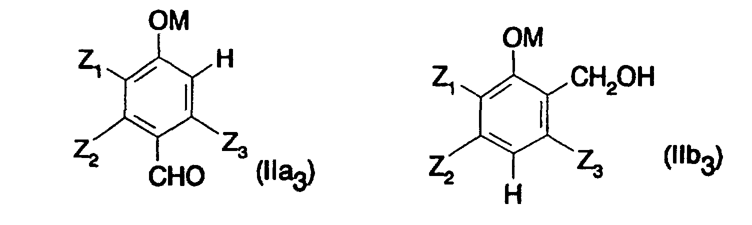 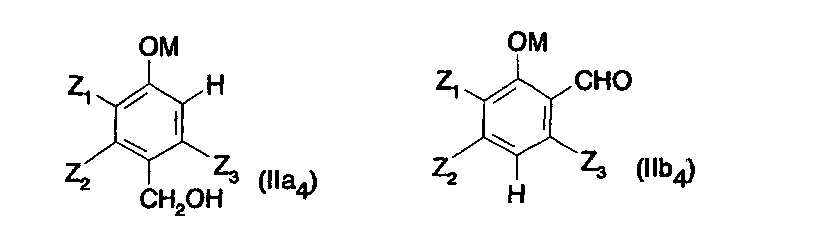 dans lesdites formules:
- M représente un atome d'hydrogène et/ou un cation métallique du groupe (la) ou (IIa) ou un cation ammonium,
- Z₁, Z₂, Z₃, ont les significations données dans les revendications 2 à 4.

19. Procédé selon la revendication 18 **caractérisé par le fait qu'**un mélange de formule (IIa₁) et (IIb₁) est obtenu par un procédé d'hydroxyméthylation d'un phénol par condensation de celui-ci avec du formaldéhyde ou d'un composé générateur de formaldéhyde en phase aqueuse en présence d'une base alcaline ou alcalino-terreuse.

20. Procédé selon la revendication 19 **caractérisé par le fait que** le phénol de départ est un phénol non substitué sur les positions ortho et para par rapport au groupe hydroxyle, de formule générale (I) : 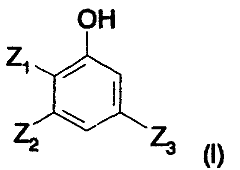 dans laquelle Z₁, Z₂, Z₃, ont les significations données précédemment.

21. Procédé selon la revendication 20 **caractérisé par le fait que** le phénol de formule (I) est le phénol, la pyrocatéchine, le gaïacol, le guétol, le 3-méthoxyphénol, le 3-éthoxyphénol, le 3-isopropoxyphénol, le 3-t-butoxyphénol, le m-crésol, l'o-crésol.

22. Procédé selon la revendication 19 **caractérisé par le fait que** l'on met en oeuvre le formaldéhyde ou tout générateur de formaldéhyde de préférence, le trioxane ou le paraformaldéhyde utilisé sous la forme de polyformaldéhydes linéaires de degré de polymérisation indifférent, ayant de préférence un nombre de motifs (CH₂O) compris entre 8 et 100 motifs.

23. Procédé selon la revendication 19 **caractérisé par le fait que** le rapport molaire formaldéhyde/phénol peut varier entre 0,5 et 2,0 et de préférence entre 0,5 et 1,5.

24. Procédé selon la revendication 19 **caractérisé par le fait que** la quantité de base présente dans le milieu d'hydroxyméthylation exprimée par le nombre de moles de base/hydroxyle phénolique du phénol à hydroxyméthyler varie entre 0,1 et 2 et, de préférence, entre 0,5 et 1,1.

25. Procédé selon la revendication 19 **caractérisé par le fait que** la température d'hydroxyméthylation est comprise entre 0 et 100°C et de préférence entre 20 et 70°C.

26. Procédé selon la revendication 19 **caractérisé par le fait que** l'on charge dans l'appareillage le phénol et le formaldéhyde, éventuellement une base, puis l'on porte le mélange réactionnel sous agitation à la température désirée, pendant la durée nécessaire à l'achèvement de la réaction jusqu'à obtention d'un mélange de composés phénoliques de formule (IIa₁) et (IIb₁).

27. Procédé selon la revendication 19 **caractérisé par le fait qu'**un mélange de composés de formule (IIa₂) et (IIb₂) est obtenu par oxydation des composés phénoliques hydroxyméthylés de formule (IIa₁ ) et (IIb₁), par l'oxygène moléculaire ou un gaz qui en contient, en phase aqueuse alcaline en présence d'un catalyseur à base d'un métal du groupe 8 de la classification périodique, de préférence, le platine et le palladium, contenant éventuellement à titre d'activateur des métaux tels que le cadmium, le cérium, le bismuth, le plomb, l'argent, le tellure ou l'étain.

28. Procédé selon la revendication 27 **caractérisé par le fait que** le pH de la solution est porté à une valeur comprise entre 8 et 13 par addition éventuelle d'une base alcaline ou alcalino-terreuse.

29. Procédé selon la revendication 27 **caractérisé par le fait que** la température de la réaction d'oxydation se situe entre 10°C et 60°C, et de préférence entre 20°C et 50°C.

30. Procédé selon la revendication 18 **caractérisé par le fait qu'**un mélange de composés phénoliques de formule (IIa₂) et (IIb₂) est obtenu par un procédé en deux étapes comprenant :
- l'hydroxyméthylation d'un phénol en milieu aqueux en présence d'une base alcaline ou alcalino-terreuse par le formaldéhyde ou un composé générateur de formaldéhyde, conduisant à un mélange de composés phénoliques hydroxyméthylés, l'un hydroxyméthylé en position 2 et l'autre en position 4,
- et l'oxydation, sans séparation intermédiaire, des composés phénoliques obtenus par l'oxygène moléculaire ou un gaz qui en contient, en phase aqueuse alcaline en présence d'un catalyseur à base d'un métal du groupe 8 de la classification périodique, éventuellement à titre d'activateur, un métal tel que ceux cités précédemment.

31. Procédé de préparation d'un 4-hydroxybenzaldéhyde **caractérisé par le fait que** l'on effectue sa séparation à partir d'un mélange d'un acide 2-hydroxybenzoïque et d'un 4-hydroxybenzaldéhyde obtenu selon l'une des revendications 1 à 30.

32. Procédé selon la revendication 31 **caractérisé par le fait que** le 4-hydroxybenzaldéhyde répondant à la formule générale : 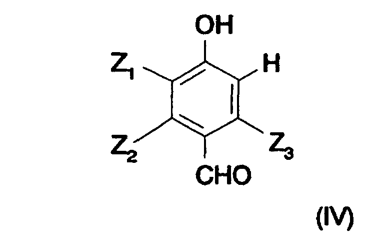 dans laquelle Z₁, Z₂, Z₃, ont les significations données précédemment,

33. Procédé selon l'une des revendications 31 et 32 **caractérisé par le fait que** l'on effectue une extraction dans un solvant organique du 4-hydroxybenzaldéhyde à pH contrôlé puis séparation du 4-hydroxybenzaldéhyde à partir de la phase organique obtenue.

34. Procédé selon la revendication 33 **caractérisé par le fait que** le solvant d'extraction est choisi parmi les cétones, de préférence, la méthyléthylcétone, la méthylisobutylcétone, la cyclohexanone ; les esters de préférence, l'acétate d'éthyle, l'acétate d'isopropyle, l'acétate de butyle ; les éther-oxydes de préférence, le diéthyléther, le diisopropyléther, le méthyl-tert-butyléther, l'éthyl-tert-butyléther, le di-n-butyléther ; les alcools lourds de préférence, le butanol, l'hexanol, l'octanol, le cyclohexanol ; les hydrocarbures aliphatiques de préférence, le n-pentane, l'hexane, l'heptane et le cyclohexane ; les hydrocarbures aliphatiques halogénés de préférence, le dichlorométhane, le dichloroéthane ; les hydrocarbures aromatiques de préférence, le toluène, les xylènes ; les hydrocarbures aromatiques halogénés de préférence, le monochlorobenzène, le dichlorobenzène et leurs mélanges.

35. Procédé selon la revendication 33 **caractérisé par le fait que** l'on ramène le pH entre 4 et 9, avant l'addition du solvant ou simultanément, par addition d'un acide protonique d'origine minérale, de préférence l'acide chlorhydrique ou l'acide sulfurique et que l'on sépare les phases aqueuse et organique.

36. Procédé selon l'une des revendications 31 et 32 **caractérisé par le fait que** l'on soumet le mélange obtenu selon l'une des revendications 1 à 30 à une décarboxylation de l'acide 2-hydroxybenzoïque partiellement ou totalement salifié par addition d'une solution d'un acide protonique et conduisant à un milieu bi-phasique comprenant en phase organique le 4-hydroxybenzaldéhyde à partir de laquelle il est séparé.

37. Procédé selon la revendication 36 **caractérisé par le fait que** l'acide 2-hvdroxybenzoïque répond à la formule générale (III) : 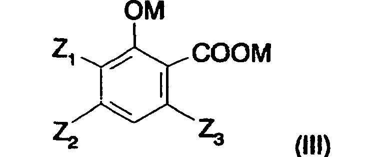 dans ladite formule (III) :
- M représente un atome d'hydrogène et/ou un cation métallique du groupe (la) ou (IIa) ou un cation ammonium,
- Z₁, Z₂, Z₃, ont les significations données dans les revendications 2 à 4.

38. Procédé selon l'une des revendications 36 et 37 **caractérisé par le fait que** l'on effectue la décarboxylation dudit acide par addition dans le milieu réactionnel, d'un acide protonique d'origine minérale, de préférence l'acide chlorhydrique ou l'acide sulfurique ou organique, jusqu'à obtention d'un pH inférieur ou égal à 3.

39. Procédé selon l'une des revendications 36 à 38 **caractérisé par le fait que** l'on chauffe le milieu réactionnel à une température variant entre 120°C et 350°C et, de préférence, de 150°C et 220°C et après refroidissement que l'on sépare le 4-hydroxybenzaldéhyde répondant préférentiellement à la formule (IV).

40. Procédé selon la revendication 39 **caractérisé par le fait que** l'on recycle le composé phénolique répondant préférentiellemnt à la formule (I), obtenu suite à la décarboxylation.

41. Procédé de préparation de la vanilline selon l'une des revendications 1 à 40 **caractérisé par le fait que** l'on soumet un mélange de composés phénoliques, o-hydroxyméthylgaïacol (A) et p-hydroxyméthylgaïacol (B) à une oxydation sélective du groupe hydroxyméthyle en position 2 du composé (A) en groupe carboxylique, et du groupe hydroxyméthyle du composé (B) en position 4 en groupe formyle, conduisant ainsi à un mélange d'un acide 2-hydroxy-3-méthoxybenzoïque et de vanilline puis à récupérer cette dernière.

42. Procédé de, préparation de vanilline selon l'une des revendications 1 à 40 **caractérisé par le fait que** l'on soumet un mélange de composés phénoliques, o-formylgaïacol (A) et p-formylgaïacol (B) à une oxydation sélective du groupe formyle en position 2 du composé (A) en groupe carboxylique, conduisant ainsi à un mélange d'un acide 2-hydroxy-3-méthoxybenzoïque et de vanilline puis à récupérer cette dernière.

43. Procédé de préparation d'éthylvanilline selon l'une des revendications 1 à 40 **caractérisé par le fait que** l'on soumet un mélange de composés phénoliques, o-hydroxyméthylguétol (A) et p-hydroxyméthylguétol (B) à une oxydation sélective du groupe hydroxyméthyle en position 2 du composé (A) en groupe carboxylique, et du groupe hydroxyméthyle du composé (B) en position 4 en groupe formyle, conduisant ainsi à un mélange d'un acide 2-hydroxy-3-éthoxybenzoïque et d'éthylvanilline puis à récupérer cette dernière.

44. Procédé de préparation d'éthylvanilline selon l'une des revendications 1 à 40 **caractérisé par le fait que** l'on soumet un mélange de composés phénoliques, o-formylguétol (A) et p-formylguétol (B) à une oxydation sélective du groupe formyle en position 2 du composé (A) en groupe carboxylique, conduisant ainsi à un mélange d'un acide 2-hydroxy-3-éthoxybenzoïque et d'éthylvanilline puis à récupérer cette dernière.

45. Utilisation du procédé décrit dans l'une des revendications 1 à 40 pour la préparation de la vanilline et de l'éthylvanilline

## Patentansprüche

1. Verfahren zur Herstellung einer 2-Hydroxybenzoesäure und eines 4-Hydroxybenzaldehyds und deren Derivaten, **dadurch gekennzeichnet, daß** man eine Mischung von Phenolverbindungen, von denen eine (A) Träger einer Formyl- oder Hydroxymethylgruppe in 2-Position und die andere (B) Träger einer Formyl- oder Hydroxymethylgruppe in 4-Position ist, einer selektiven Oxidation der Formyl- oder Hydroxymethylgruppe in 2-Position der Verbindung (A) zu einer Carboxylgruppe und gegebenenfalls einer Hydroxymethylgruppe in 4-Position der Verbindung (B) zu einer Formylgruppe in Gegenwart eines Katalysators auf Basis von Palladium und/oder Platin und einer Base, welche in einer derartigen Menge eingesetzt wird, daß das Verhältnis zwischen der Molanzahl der basischen Verbindung und der Molanzahl der Phenolverbindungen 2 bis 10 beträgt, unterzieht, was zu einer Mischung einer 2-Hydroxybenzoesäure und eines 4-Hydroxybenzaldehyds führt.

2. Verfahren nach Anspruch 1, **dadurch gekennzeichnet, daß** die Mischung der eingesetzten Phenolverbindungen der allgemeinen Formel (II) entspricht: 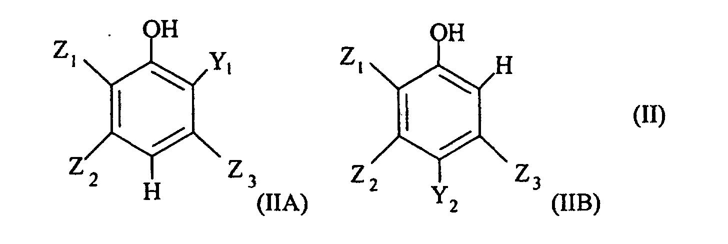 wobei in den Formeln (IIA) und (IIB):
- Y₁ und Y₂, identisch oder verschieden, eine der nachfolgenden Gruppen darstellen:
• eine Gruppe -CHO,
• eine Gruppe -CH₂OH,
- Z₁, Z₂ und Z₃, identisch oder verschieden, ein Wasserstoffatom, eine Alkyl-, Alkenyl-, Alkoxy-, Hydroxyalkyl-, Alkoxyalkyl-, Cycloalkyl-, oder eine Arylgruppe, eine Hydroxylgruppe, eine Nitrogruppe, ein Halogenatom oder eine Trifluormethylgruppe darstellen.

3. Verfahren nach einem der Ansprüche 1 und 2, **dadurch gekennzeichnet, daß** die Phenolverbindungen der Formel (IIA) und (IIB) entsprechen, wobei Z₁, Z₂ und Z₃, identisch oder verschieden, eines oder eine der folgenden Atome oder Gruppen darstellen:
• ein Wasserstoffatom,
• einen linearen oder verzweigten Alkylrest mit 1 bis 12 Kohlenstoffatomen, vorzugsweise 1 bis 4 Kohlenstoffatomen, vorzugsweise Methyl, Ethyl, Propyl, Isopropyl, Butyl, Isobutyl, sek.-Butyl oder tert.-Butyl,
• einen linearen oder verzweigten Alkenylrest mit 2 bis 12 Kohlenstoffatomen, vorzugsweise 2 bis 4 Kohlenstoffatomen, vorzugsweise Vinyl oder Allyl,
• einen linearen oder verzweigten Alkoxyrest mit 1 bis 12 Kohlenstoffatomen, vorzugsweise mit 1 bis 4 Kohlenstoffatomen, vorzugsweise Methoxy, Ethoxy, Propoxy, Isopropoxy, Butoxy, Isobutoxy, sek.-Bu toxy oder tert.-Butoxy,
• einen Phenylrest,
• ein Halogenatom, vorzugsweise ein Fluor-, Chlor- oder Bromatom.

4. Verfahren nach einem der Ansprüche 1 bis 3, **dadurch gekennzeichnet, daß** die Phenolverbindungen den Formeln (IIA) und (IIB) entsprechen, wobei Z₁ ein Wasserstoffatom oder einen linearen oder verzweigten Alkyl- oder Alkoxyrest mit 1 bis 6 Kohlenstoffatomen, vorzugsweise 1 bis 4 Kohlenstoffatomen, darstellt; Z₂ und Z₃ ein Wasserstoffatom darstellen; Y₁ und Y₂, identisch, eine Formylgruppe oder eine Hydroxymethylgruppe darstellen.

5. Verfahren nach einem der Ansprüche 1 bis 4, **dadurch gekennzeichnet, daß** die der Formel (II) entsprechende Mischung von Phenolverbindungen darstellt:
- ortho-Hydroxymethylphenol und para-Hydroxymethylpheriol,
- ortho-Hydroxymethylguajakol und para-Hydroxymethylguajakol,
- ortho-Formylguajakol und para-Formylguajakol,
- ortho-Hydroxymethylguetol und para-Hydroxymethylguetol,
- ortho-Formylguetol und para-Formylguetol.

6. Verfahren nach einem der Ansprüche 1 bis 5, **dadurch gekennzeichnet, daß** man die Mischung von Phenolverbindungen der Formel (II) in flüssiger Phase mit Hilfe von molekularem Sauerstoff oder einem Sauerstoff enthaltenden Gas in einem wäßrigen Milieu, das eine basische Verbindung enthält, in Gegenwart eines Katalysators auf Basis von Platin und/oder Palladium oxidiert, wobei der Katalysator gegebenenfalls als Aktivierungsmittel Metalle, ausgewählt aus Cadmium, Cer, Wismuth, Blei, Silber, Tellur oder Zinn, enthält.

7. Verfahren nach Anspruch 6, **dadurch gekennzeichnet, daß** der Platin- und/oder Palladiumkatalysator in Form von Platinmohr (Platinruß), Palladiummohr (Palladiumruß), Platinoxid, Palladiumoxid oder des Edelmetalls als solchen, aufgebracht auf verschiedenen Trägem, wie Kohlenstoffruß (Rußschwarz, Carbon Black), Calciumcarbonat, aktivierten Aluminiumoxiden und Siliciumoxiden oder entsprechenden Materialien, vorzugsweise Kohlenstoffruß (Rußschwarz, Carbon Black), bereitgestellt wird.

8. Verfahren nach einem der Ansprüche 6 und 7, **dadurch gekennzeichnet, daß** die Menge an einzusetzendem Katalysator, berechnet als Gewicht des Metalls, bezogen auf das Gewicht der Mischung von Phenolverbindungen der Formel (II), von 0,01 bis 10 %, vorzugsweise 0,04 bis 2 %, variieren kann.

9. Verfahren nach einem der Ansprüche 6 bis 8, **dadurch gekennzeichnet, daß** das Aktivierungsmittel ein organisches oder anorganisches Wismuthderivat, ausgewählt aus der Gruppe von Wismuthoxiden; Wismuthhydroxyden; Wismuth- oder Wismuthylsalzen anorganischer Wasserstoffsäuren, vorzugsweise Chloriden, Bromiden, Iodiden, Sulfiden, Seleniden und Telliden; Wismuth- oder Wismuthylsalzen anorganischer Oxysäuren (Sauerstoffsäuren), vorzugsweise Sulfit, Sulfat, Nitrit, Nitrat, Phosphit, Phosphat, Pyrophosphat, Carbonat, Perchlorat, Antimonat, Arsenat, Selenit, Selenat; Wismuth- oder Wismuthylsalzen aliphatischer oder aromatischer organischer Säuren, vorzugsweise Acetat, Propionat, Salicylat, Benzoat, Oxalat, Tartrat, Lactat, Citrat; Wismuth- oder Wismuthylphenaten, vorzugsweise Gallaten und Pyrogallaten.

10. Verfahren nach Anspruch 9, **dadurch gekennzeichnet, daß** das Wismuthderivat ausgewählt ist aus der Gruppe von Wismuthoxiden Bi₂O₃ und Bi₂O₄; Wismuthhydroxid Bi(OH)₃; Wismuthchlorid BiCl₃; Wismuthbromid BiBr₃; Wismuthiodid BiI₃; neutralem Wismuthsulfat Bi₂(SO₄)₃; neutralem Wismuthnitrat Bi(NO₃)₃ · 5 H₂O; Wismuthylnitrat BiO(NO₃); Wismuthylcar- bonat (BiO)₂CO₃ · 0,5 H₂O; Wismuthacetat Bi(C₂H₃O₂)₃; Wismuthylsalicylat C₆H₄CO₂(BiO)OH.

11. Verfahren nach einem der Ansprüche 6 bis 10, **dadurch gekennzeichnet, daß** die Menge an Aktivierungsmittel derart ausgewählt wird, daß hierdurch einerseits mindestens 0,1 Gew.-% Metallaktivierungsmittel, bezogen auf das Gewicht des eingesetzten Metalls M₁, und andererseits 10 bis 900 Gew.-ppm Aktivierungsmittel, bezogen auf die Mischung der Phenolverbindungen der Formel (II), in dem Milieu bereitgestellt werden.

12. Verfahren nach einem der Ansprüche 6 bis 11, **dadurch gekennzeichnet, daß** die Oxidationsreaktion in einem Temperaturbereich von 30 °C bis 200 °C, vorzugsweise von 40 °C bis 160 °C, durchgeführt wird.

13. Verfahren nach einem der Ansprüche 6 bis 12, **dadurch gekennzeichnet, daß** man unter einem Druck von 1 bis 20 bar verfährt.

14. Verfahren nach einem der Ansprüche 1 bis 13, **dadurch gekennzeichnet, daß** die Oxidation in einem wäßrigen Milieu durchgerührt wird, das eine gelöste basische Verbindung, vorzugsweise Natrium- oder Kaliumhydroxid, in einer Menge von 2 bis 10, vorzugsweise 2 bis 4 mol, anorganische Base pro mol der Phenolverbindungen der Formel (II) enthält.

15. Verfahren nach einem der Ansprüche 1 bis 14, **dadurch gekennzeichnet, daß** die Temperatur 30 °C bis 200 °C, vorzugsweise 40 °C bis 160 °C, beträgt.

16. Verfahren nach Anspruch 15, **dadurch gekennzeichnet, daß** im Falle eines Platinkatalysators die Temperatur 100 °C bis 160 °C beträgt.

17. Verfahren nach Anspruch 15, **dadurch gekennzeichnet, daß** im Falle eines Palladiumkatalysators die Temperatur 30 °C bis 200 °C, vorzugsweise 30 °C bis 150 °C, beträgt, wobei die Menge an eingesetzter Base 2 bis 4 mol pro mol der Phenolverbindungen der Formel (II) beträgt.

18. Verfahren nach einem der Ansprüche 1 bis 17, **dadurch gekennzeichnet, daß** die Ausgangssubstrate den folgenden Formeln entsprechen: 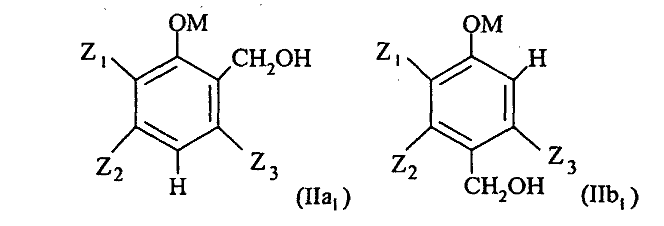 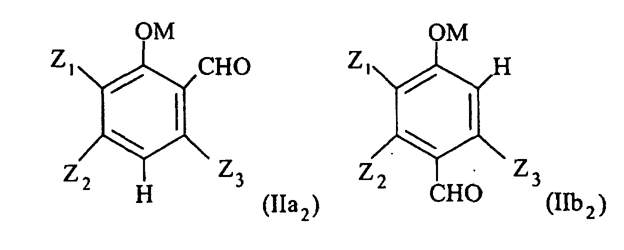 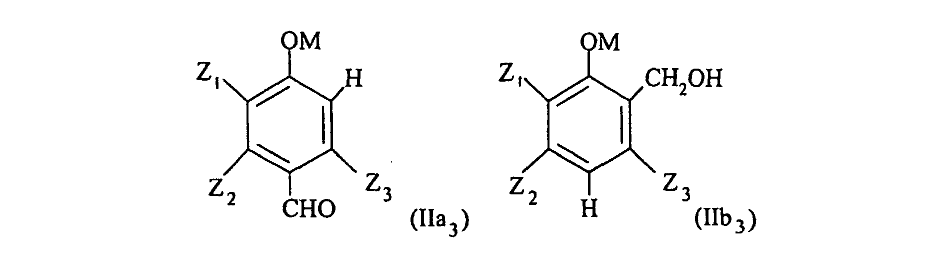 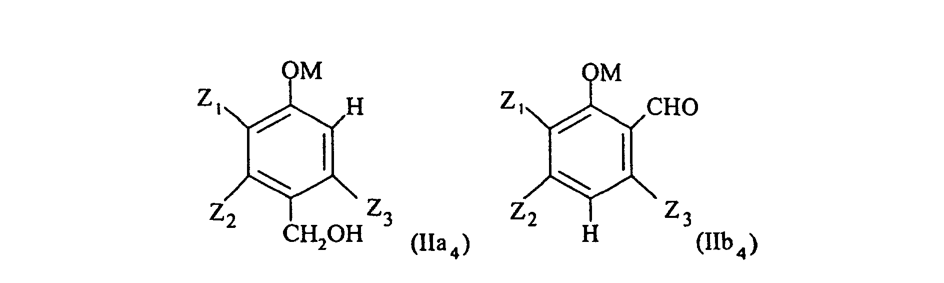 wobei in den Formeln:
- M ein Wasserstoffatom und/oder ein metallisches Kation aus der Gruppe (Ia) oder (IIa) oder ein Ammoniumkation darstellt,
- Z₁, Z₂ und Z₃ die in den Ansprüchen 2 bis 4 angegebenen Bedeutungen haben.

19. Verfahren nach Anspruch 18, **dadurch gekennzeichnet, daß** die Mischung der Formel (IIa₁) und (IIb₁) durch ein Hydroxymethylierungsverfahren eines Phenols durch Kondensation des Phenols mit Formaldehyd oder mit einer Verbindung, die Formaldehyd generieren kann, in wäßriger Phase in Gegenwart einer Alkali- oder Erdalkalimetallbase erhalten wird.

20. Verfahren nach Anspruch 19, **dadurch gekennzeichnet, daß** das Ausgangsphenol ein in den ortho- und para-Positionen, bezogen auf die Hydroxylgruppe, nichtsubstituiertes Phenol der allgemeinen Formel (I) ist: 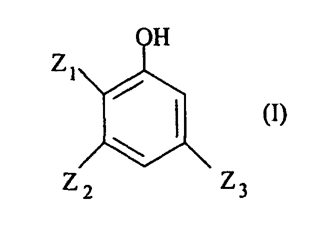 wobei Z₁, Z₂ und Z₃ die zuvor angegebenen Bedeutungen haben.

21. Verfahren nach Anspruch 20, **dadurch gekennzeichnet, daß** das Phenol der Formel (I) Phenol, Pyrocatechin, Guajakol, Guetol, 3-Methoxyphenol, 3-Ethoxyphenol, 3-Isopropoxyphenol, 3-tert.-Butoxyphenol, meta-Kresol oder ortho-Kresol ist.

22. Verfahren nach Anspruch 19, **dadurch gekennzeichnet, daß** man Formaldehyd oder jede andere Verbindung, die Formaldehyd generieren kann, vorzugsweise Trioxan oder Paraformaldehyd in Form von linearen Polyformaldehyden mit unterschiedlichem Polymerisationsgrad, vorzugsweise mit 8 bis 100 (CH₂O)-Einheiten, einsetzt.

23. Verfahren nach Anspruch 19, **dadurch gekennzeichnet, daß** das Formaldehyd/Phenol-Molverhältnis von 0,5 bis 2,0, vorzugsweise von 0,5 bis 1,5, variieren kann.

24. Verfahren nach Anspruch 19, **dadurch gekennzeichnet, daß** die Menge an in dem Hydroxymethylierungsmilieu vorhandener Base, berechnet als Anzahl der Mol an Base/phenolisches Hydroxyl des zu hydroxymethylierenden Phenols, von 0,1 bis 2, vorzugsweise von 0,5 bis 1,1, variiert.

25. Verfahren nach Anspruch 19, **dadurch gekennzeichnet, daß** die Hydroxymethylierungstemperatur 0 bis 100 °C, vorzugsweise 20 bis 70 °C, beträgt.

26. Verfahren nach Anspruch 19, **dadurch gekennzeichnet, daß** man die Apparatur mit Phenol, Formaldehyd und gegebenenfalls einer Base belädt und man anschließend die Reaktionsmischung während der Zeitdauer, die zur Vollendung der Reaktion bis zum Erhalt einer Mischung der Phenolverbindungen der Formel (IIa₁) und (IIb₁) erforderlich ist, unter Rühren auf die gewünschte Temperatur bringt.

27. Verfahren nach Anspruch 19, **dadurch gekennzeichnet, daß** eine Mischung der Verbindungen der Formel (IIa₂) und (IIb₂) durch Oxidation der hydroxymethylierten Phenolverbindungen der Formel (IIa₁) und (IIb₁) mit Hilfe von molekularem Sauerstoff oder einem Sauerstoff enthaltenden Gas in wäßriger alkalischer Phase in Gegenwart eines Katalysators auf Basis eines Metalls der Gruppe 8 des Periodensystems, vorzugsweise Platin und Palladium, erhalten wird, wobei der Katalysator gegebenenfalls als Aktivierungsmittel Metalle, wie Cadmium, Cer, Wismuth, Blei, Silber, Tellur oder Zinn, enthält.

28. Verfahren nach Anspruch 27, **dadurch gekennzeichnet, daß** der pH-Wert der Lösung auf einen Wert zwischen 8 und 13, gegebenenfalls durch Zugabe einer Alkali- oder Erdalkalibase, gebracht wird.

29. Verfahren nach Anspruch 27, **dadurch gekennzeichnet, daß** die Temperatur der Oxidationsreaktion 10 °C bis 60 °C, vorzugsweise 20 °C bis 50 °C, beträgt.

30. Verfahren nach Anspruch 18, **dadurch gekennzeichnet, daß** eine Mischung von Phenolverbindungen der Formel (IIa₂) und (IIb₂) durch ein zweistufiges Verfahren erhalten wird, welches umfaßt:
- Hydroxymethylierung eines Phenols in wäßrigem Milieu in Gegenwart einer Alkali- oder Erdalkalimetallbase durch Formaldehyd oder eine Verbindung, welche Formaldehyd generieren kann, was zu einer Mischung von hydroxymethylierten Phenolverbindungen führt, von denen die eine in 2-Position und die andere in 4-Position hydroxymethyliert ist,
- und, ohne zwischenzeitliche Trennung, Oxidation der erhaltenen Phenolverbindungen mit Hilfe von molekularem Sauerstoff oder einem Sauerstoff enthaltenden Gas in wäßriger alkalischer Phase in Gegenwart eines Katalysators auf Basis eines Metalls der Gruppe 8 des Periodensystems, gegebenenfalls mit einem wie zuvor beschriebenen Metall als Aktivierungsmittel.

31. Verfahren zur Herstellung eines 4-Hydroxybenzaldehyds, **dadurch gekennzeichnet, daß** man seine Trennung ausgehend von einer Mischung einer 2-Hydroxybenzoesäure und eines 4-Hydroxybenzaldehyds, erhalten nach einem der Ansprüche 1 bis 30, durchführt.

32. Verfahren nach Anspruch 31, **dadurch gekennzeichnet, daß** der 4-Hydroxybenzaldehyd der allgemeinen Formel entspricht: 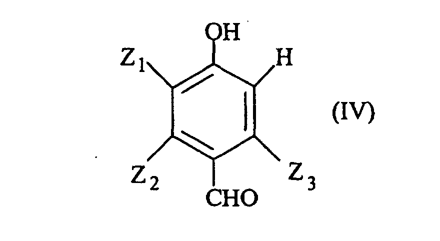 wobei Z₁, Z₂ und Z₃ die zuvor angegebenen Bedeutungen haben.

33. Verfahren nach einem der Ansprüche 31 und 32, **dadurch gekennzeichnet, daß** man in einem organischen Lösemittel eine Extraktion des 4-Hydroxybenzaldehyds bei einem kontrollierten pH-Wert durchführt und man anschließend den 4-Hydroxybenzaldehyd ausgehend von der erhaltenen organischen Phase abtrennt.

34. Verfahren nach Anspruch 33, **dadurch gekennzeichnet, daß** das Extraktionslösemittel ausgewählt ist aus Ketonen, vorzugsweise Methylethylketon, Methylisobutylketon, Cyclohexanon; Estern, vorzugsweise Ethylacetat, Isopropylacetat, Butylacetat; Etheroxiden, vorzugsweise Diethylether, Diisopropylether, Methyl-tert.-butylether, Ethyl-tert.-butylether, Di-n-butylether; schweren Alkoholen, vorzugsweise Butanol, Hexanol, Octanol, Cyclohexanol; aliphatischen Kohlenwasserstoffen, vorzugsweise n-Pentan, Hexan, Heptan und Cyclohexan; halogenierten aliphatischen Kohlenwasserstoffen, vorzugsweise Dichlormethan und Dichlorethan; aromatischen Kohlenwasserstoffen, vorzugsweise Toluol und Xylolen; halogenierten aromatischen Kohlenwasserstoffen, vorzugsweise Monochlorbenzol, Dichlorbenzol; und deren Mischungen.

35. Verfahren nach Anspruch 33, **dadurch gekennzeichnet, daß** man den pH-Wert vor Zugabe des Lösemittels oder gleichzeitig hiermit durch Zugabe einer Protonensäure anorganischen Ursprungs, vorzugsweise Chlorwasserstoffsäure oder Schwefelsäure, wieder auf einen Wert von 4 bis 9 bringt und man die wäßrige und organische Phase trennt.

36. Verfahren nach einem der Ansprüche 31 und 32, **dadurch gekennzeichnet, daß** man die nach einem der Ansprüche 1 bis 30 erhaltene Mischung einer Decarboxylierung der teilweise oder vollständig als Salz vorliegenden 2-Hydroxybenzoesäure durch Zugabe einer Lösung einer Protonensäure unterzieht, was zu einem Zweiphasenmilieu führt, welches in der organischen Phase den 4-Hydroxybenzaldehyd enthält, von welcher er abgetrennt wird.

37. Verfahren nach Anspruch 36, **dadurch gekennzeichnet, daß** die 2-Hydroxybenzoesäure der allgemeinen Formel (III) entspricht: 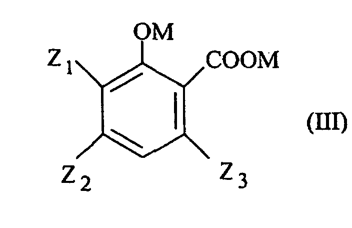 wobei in der Formel (III):
- M ein Wasserstoffatom und/oder ein Metallkation der Gruppe (Ia) oder (IIa) oder ein Ammoniumkation darstellt,
- Z₁, Z_{z} und Z₃ die in den Ansprüchen 2 bis 4 angegebenen Bedeutungen haben.

38. Verfahren nach einem der Ansprüche 36 und 37, **dadurch gekennzeichnet, daß** man die Decarboxylierung der Säure durch Zugabe einer Protonensäure anorganischen Ursprungs, vorzugsweise Chlorwasserstoffsäure oder Schwefelsäure, oder organischen Ursprungs bis zum Erhalt eines pH-Werts kleiner oder gleich 3 durchführt.

39. Verfahren nach einem der Ansprüche 36 bis 38, **dadurch gekennzeichnet, daß** man das Reaktionsmilieu auf eine Temperatur von 120 °C bis 350 °C, vorzugsweise 150 °C bis 220 °C, erwärmt und man nach Abkühlung den 4-Hydroxybenzaldehyd, welcher vorzugsweise der Formel (IV) entspricht, abtrennt.

40. Verfahren nach Anspruch 39, **dadurch gekennzeichnet, daß** man die nach der Decarboxylierung erhaltene, vorzugsweise der Formel (I) entsprechende Phenolverbindung rezykliert.

41. Verfahren zur Herstellung von Vanillin nach einem der Ansprüche 1 bis 40, **dadurch gekennzeichnet, daß** man eine Mischung von Phenolverbindungen, ortho-Hydroxymethylguajakol (A) und para-Hydroxymethylguajakol (B) einer selektiven Oxidation der Hydroxymethylgruppe in 2-Position der Verbindung (A) zu einer Carboxylgruppe und der Hydroxymethylgruppe in 4-Position der Verbindung (B) zu einer Formylgruppe unterzieht, was zu einer Mischung einer 2-Hydroxy-3-methoxybenzoesäure und Vanillin führt, und man anschließend letzteres erhält bzw. gewinnt.

42. Verfahren zur Herstellung von Vanillin nach einem der Ansprüche 1 bis 40, **dadurch gekennzeichnet, daß** man eine Mischung von Phenolverbindungen, ortho-Formylguajakol (A) und para-Formylguajakol (B) einer selektiven Oxidation der Formylgruppe in 2-Position der Verbindung (A) zu einer Carboxylgruppe unterzieht, was zu einer Mischung einer 2-Hydroxy-3-methoxybenzoesäure und Vanillin führt, und man anschließend letzteres erhält bzw. gewinnt.

43. Verfahren zur Herstellung von Ethylvanillin nach einem der Ansprüche 1 bis 40, **dadurch gekennzeichnet, daß** man eine Mischung von Phenolverbindungen, ortho-Hydroxymethylguetol (A) und para-Hydroxymethylguetol (B) einer selektiven Oxidation der Hydroxymethylgruppe in 2-Position der Verbindung (A) zu einer Carboxylgruppe und der Hydroxymethylgruppe in 4-Position der Verbindung (B) zu einer Formylgruppe unterzieht, was zu einer Mischung einer 2-Hydroxy-3-ethoxybenzoesäure und Ethylvanillin führt, und man anschließend letzteres erhält bzw. gewinnt.

44. Verfahren zur Herstellung von Ethylvanillin nach einem der Ansprüche 1 bis 40, **dadurch gekennzeichnet, daß** man eine Mischung von Phenolverbindungen, ortho-Formylguetol (A) und para-Formylguetol (B) einer selektiven Oxidation der Formylgruppe in 2-Position der Verbindung (A) zu einer Carboxylgruppe unterzieht, was zu einer Mischung einer 2-Hydroxy-3-ethoxybenzoesäure und Ethylvanillin führt, und man anschließend letzteres erhält bzw. gewinnt.

45. Anwendung des in einem der Ansprüche 1 bis 40 beschriebenen Verfahrens zur Herstellung von Vanillin und Ethylvanillin.

## Claims

1. A process for preparing a 2-hydroxybenzoic acid and a 4-hydroxybenzaldehyde and derivatives thereof, **characterized in that** a mixture of phenolic compounds, one (A) carrying a formyl or hydroxymethyl group in the 2 position; and the other (B) carrying a formyl or hydroxymethyl group in the 4 position, undergoes selective oxidation of the formyl or hydroxymethyl group in the 2 position of compound (A) to a carboxylic group, and optionally of a hydroxymethyl group of compound (B) in the 4 position to a formyl group, resulting in a mixture of a 2-hydroxybenzoic acid and a 4-hydroxybenzaldehyde, in the presence of a catalyst based on palladium and/or platinum and a base employed in a quantity such that the ratio between the number of moles of basic agent and the number of moles of phenolic compounds is in the range 2 to 10.

2. A process according to claim 1, **characterised in that** the mixture of phenolic compounds used has general formula (II): 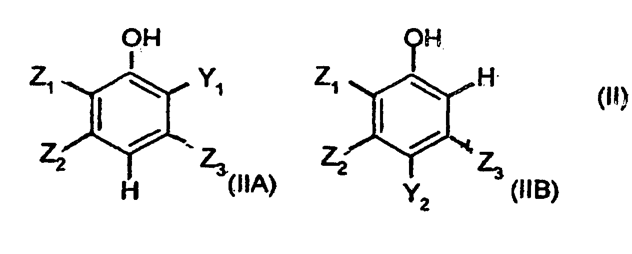 where, in said formulae (IIA) and (IIB):
- Y₁ and Y₂, which may be identical or different, represent one of the following groups:
. a -CHO group
. a -CH₂OH group,
- Z₁, Z₂ and Z₃, which may be identical or different, represent a hydrogen atom, an alkyl, alkenyl, alkoxy, hydroxyalkyl, alkoxyalkyl, cycloalkyl, or aryl radical, a hydroxyl group, a nitro group, a halogen atom, or a trifluoromethyl group.

3. A process according to claim 1 or claim 2, **characterised in that** the phenolic compounds have formula (IIA) and (IIB), where Z₁, Z₂ and Z₃, which may be identical or different, represent one of the following atoms or groups:
. a hydrogen atom,
. a linear or branched alkyl radical containing 1 to 12 carbon atoms, preferably 1 to 4 carbon atoms, preferably methyl, ethyl, propyl, isopropyl, butyl, isobutyl, sec-butyl or tert-butyl,
. a linear or branched alkenyl radical containing 2 to 12 carbon atoms, preferably 2 to 4 carbon atoms, preferably vinyl or allyl,
. a linear or branched alkoxy radical containing 1 to 12 carbon atoms, preferably 1 to 4 carbon atoms, preferably a methoxy, ethoxy, propoxy, isopropoxy, butoxy, isobutoxy, sec-butoxy or tert-butoxy radicals,
. a phenyl radical,
. a halogen atom, preferably a fluorine, chlorine or bromine atom.

4. A process according to one of claims 1 to 3, **characterised in that** the phenolic compounds have the formulae (IIA) and (IIB), where Z₁ represents a hydrogen atom or a linear or branched alkoxy radical containing 1 to 6 carbon atoms, preferably 1 to 4 carbon atoms; Z₂ and Z₃ represent a hydrogen atom; and Y₁ and Y₂ are identical and represent a formyl group or a hydroxymethyl group.

5. A process according to one of claims 1 to 4, **characterised in that** the mixture of phenolic compounds having formula (II) is:
- o-hydroxymethylphenol and p-hydroxymethylphenol,
- o-hydroxymethylguaiacol and p-hydroxymethylguaiacol,
- o-formylguaiacol and p-formylguaiacol,
- o-hydroxymethylguetol and p-hydroxymethylguetol,
- o-formylguetol and p-formylguetol.

6. A process according to one of claims 1 to 5, **characterised in that** a mixture of phenolic compounds having formula (II) is oxidised in the liquid phase using molecular oxygen or a gas containing molecular oxygen, in an aqueous medium comprising a basic agent, in the presence of a cartalyst based on platinum and/or palladium, optionally comprising, as an activator, metals selected from cadmium, cerium, bismuth, lead, silver, tellurium and tin.

7. A process according to claim 6, **characterised in that** the platinum and/or palladium catalyst is provided in the form of platinum black, palladium black, platinum oxide, palladium oxide, or the noble metal itself, deposited on different supports such as carbon black, calcium carbonate, activated aluminas and silicas, or equivalent materials, preferably carbon black.

8. A process according to claim 6 or claim 7, **characterised in that** the quantity of catalyst to be used, expressed as the weight of metal with respect to that of the phenolic compound with formula (II), is between 0.01 and 10%, preferably between 0.04 and 2%.

9. A process according to one of claims 6 to 8, **characterised in that** the activator is an inorganic or organic bismuth derivative selected from the group formed by: bismuth oxides; bismuth hydroxides; bismuth or bismuthyl salts of inorganic hydracids, preferably the chloride, bromide, iodide, sulphide, selenide or telluride; bismuth or bismuthyl salts of inorganic oxyacids. preferably the sulphite, sulphate, nitrite, nitrate, phosphite, phosphate, pyrophosphate, carbonate, perchlorate, antimonate, arsenate, selenite or selenate; bismuth or bismuthyl salts of aliphatic or aromatic organic acids, preferably the acetate, propionate, salicylate, benzoate, oxalate, tartrate, lactate, or citrate; and bismuth or bismuthyl phenates, preferably the gallate or pyrogallate.

10. A process according to claim 9, **characterised in that** the bismuth derivative is selected from the group formed by: bismuth oxides: Bi₂O₃ and Bi₂O₄; bismuth hydroxide Bi(OH)₃; bismuth chloride BiCl₃; bismuth bromide BiBr₃; bismuth iodide BiI₃; neutral bismuth sulphate Bi₂(SO₄)₃; neutral bismuth nitrate Bi(NO₃)₃,5H₂O; bismuthyl nitrite (BiO)NO₂,O.5H₂O; bismuthyl carbonate (BiO)₂CO₃,0.5H₂O; bismuth acetate Bi(C₂H₃O₂)₃ and bismuthyl salicylate C₆H₄CO₂(BiO)(OH).

11. A process according to one of claims 6 to 10, **characterised in that** the quantity of activator is selected so that the medium contains: at least 0.1% by weight of metal activator with respect to the weight of metal M₁ used, and 10 to 900 ppm by weight of activator with respect to the phenolic compounds with formula (II).

12. A process according to one of claims 6 to 11, **characterised in that** the oxidation reaction is carried out within a temperature range of 30°C to 200°C, preferably between 40°C and 160°C.

13. A process according to one of claims 6 to 12, **characterised in that** a pressure of 1 to 20 bar is used.

14. A process according to one of claims 1 to 13, **characterised in that** oxidation is carried out in an aqueous medium containing, in solution, a basic agent, preferably sodium or potassium hydroxide, in a quantity such that it represents 2 to 10 moles, preferably 2 to 4 moles, of mineral base per mole of phenolic compounds with formula (II).

15. A process according to one of claims 1 to 14, **characterised in that** the temperature is selected to be between 30°C and 200°C, preferably between 40°C and 160°C.

16. A process according to claim 15, **characterised in that** when using a platinum catalyst, the temperature is selected to be between 100°C and 160°C.

17. A process according to claim 15, **characterised in that** when using a palladium catalyst, the temperature is selected to be between 30°C and 200°C, preferably between 30°C and 150°C; and the quantity of base to be used is between 2 and 4 moles per mole of phenolic compounds with formula (II).

18. A process according to one of claims 1 to 17, **characterised in that** the starting substrates have the formulae: 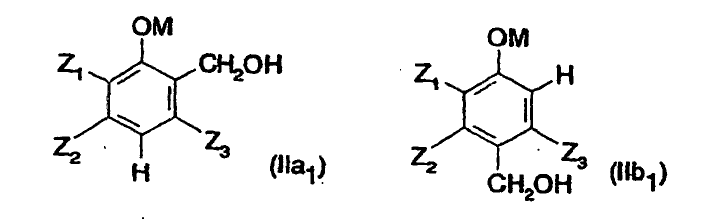 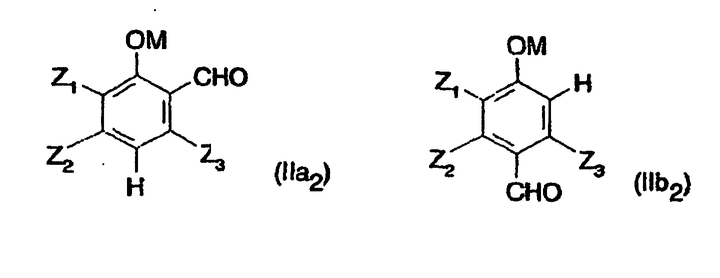 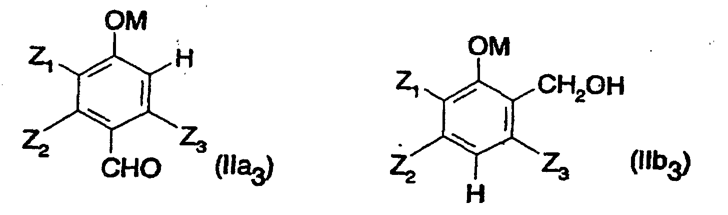 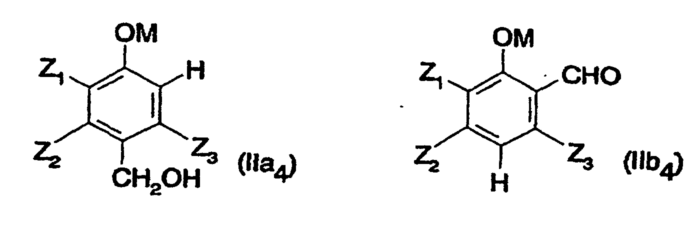 where, in said formulae:
- M represents a hydrogen atom and/or a metal cation from group (Ia) or (IIa) or an ammonium cation,
- Z₁, Z₂, Z₃ have the meanings given in claims 2 to 4.

19. A process according to claim 18, **characterised in that** a mixture of formula (IIa₁) and (IIb₁) is obtained by hydroxymethylation of a phenol by condensation thereof with formaldehyde or a formaldehyde generator in an aqueous phase in the presence of an alkaline or alkaline earth base.

20. A process according to claim 19, **characterised in that** the starting phenol is unsubstituted at the ortho and para positions with respect to the hydroxyl group, with general formula (I): 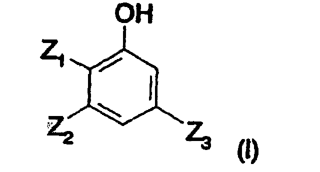 in which Z₁, Z₂, Z₃ have the meanings given above.

21. A process according to claim 20, **characterised in that** the phenol with formula (I) is phenol, pyrocatechin, guaiacol, guetol, 3-methoxyphenol, 3-ethoxyphenol, 3-isopropoxyphenol, 3-t-butoxyphenol, m-cresol or o-cresol.

22. A process according to claim 19, **characterised in that** formaldehyde or any formaldehyde generator is used, preferably trioxane or paraformaldehyde used as linear paraformaldehydes of any degree of polymerisation, preferably containing 8 to 100 (CH₂O) units.

23. A process according to claim 19, **characterised in that** the formaldehyde/phenol molar ratio can vary between 0.5 and 2.0, and preferably between 0.5 and 1.5.

24. A process according to claim 19, **characterised in that** the quantity of base present in the hydroxymethylation medium, expressed as the number of moles of base/phenolic hydroxyl group of the phenol to be hydroxymethylated, varies between 0.1 and 2, and preferably between 0.5 and 1.1.

25. A process according to claim 19, **characterised in that** the hydroxymethylation temperature is between 0 and 100°C and preferably between 20 and 70°C.

26. A process according to claim 19, **characterised in that** the phenol and formaldehyde, and any base, are placed in the apparatus, then the reaction mixture is heated with stirring to the desired temperature for the time required to bring the reaction to completion to obtain a mixture of phenolic compounds with formulae (Ha₁) and (IIb₁).

27. A process according to claim 19, **characterised in that** a mixture of compounds with formulae (IIa₂) and (IIb₂) is obtained by oxidising hydroxymethylated phenolic compounds with formulae (Ha₁) and (IIb₁), using molecular oxygen or a gas containing molecular oxygen in an alkaline aqueous phase in the presence of a catalyst based on a metal from group 8 of the periodic classification, preferably platinum and palladium, optionally containing metals such as cadmium, cerium, bismuth, lead, silver, tellurium or tin as an activator.

28. A process according to claim 27, **characterised in that** the pH of the solution is brought to a value between 8 and 13 by optional addition of an alkaline or alkaline earth base.

29. A process according to claim 27, **characterised in that** the temperature of the oxidation reaction is between 10°C and 60°C, and preferably between 20°C and 50°C.

30. A process according to claim 18, **characterised in that** a mixture of phenolic compounds with formulae (IIa₂) and IIb₂) is obtained by a two-step process comprising:
- hydroxymethylation of a phenol in an aqueous medium in the presence of an alkaline or alkaline-earth base by formaldehyde or a formaldehyde generator producing a mixture of hydroxymethylated phenolic compounds, one hydroxymethylated in the 2 position, the other in the 4 position,
- and oxidation, without intermediate separation, of phenolic compounds obtained by molecular oxygen or a gas containing molecular oxygen in an alkaline aqueous phase in the presence of a catalyst based on a metal from group 8 of the periodic classification, possibly as an activator, this metal being such as those cited above.

31. A process for preparing a 4-hydroxybenzaldehyde, **characterized in that** separation is carried out from a mixture of a 2-hydroxybenzoic acid and a 4-hydroxybenzaldehyde obtained according to one of claims 1 to 30.

32. A process according to claim 31, **characterized in that** the 4-hydroxybenzaldehyde has general formula: 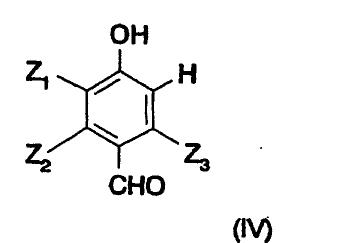 where Z₁, Z₂ and Z₃ have the meanings given above.

33. A process according to claim 31 or claim 32, **characterized in that** the 4-hydroxybenzaldehyde is extracted in an organic solvent at a controlled pH followed by separation of the 4-hydroxybenzaldehyde from the organic phase obtained.

34. Process according to claim 33 **characterised in that** the extraction solvent is selected from ketones, preferably methylethylketone, methylisobutylketone, cylcohexanone; esters, preferably ethyl acetate, isopropyl acetate, butyl acetate; ether oxides, preferably diethylether, diisopropylether, methyl-tert-butylether, ethyl-tert-butylether, di-n-butylether; heavy alcohols, preferably butanol, hexanol, octanol, cyclohexanol; aliphatic hydrocarbons, preferably n-pentane, hexane, heptane and cyclohexane; halogenated aliphatic hydrocarbons, preferably dichloromethane, dichloroethane; aromatic hydrocarbons, preferably toluene, xylenes; and halogenated aromatic hydrocarbons, preferably monochlorobenzene, dichlorobenzene, and mixtures thereof.

35. A process according to claim 33, **characterised in that** the pH is returned to between 4 and 9 prior to addition of the solvent or simultaneously by adding a protonic acid of mineral origin, preferably hydrochloric acid or sulphuric acid, and that the aqueous and organic phases are separated.

36. A process according to claim 31 or claim 32, **characterized in that** the mixture obtained in accordance with one of claims 1 to 30 undergoes decarboxylation of the 2-hydroxybenzoic acid, partially or completely in its salt form, by adding a solution of a protonic acid and resulting in a two-phase medium comprising the 4-hydroxybanzaldehyde in the organic phase from which it is separated.

37. A process according to claim 36, **characterized in that** the acids to be decarboxylated have the following formula: 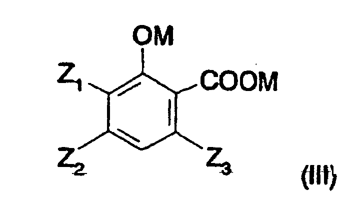 where, in said formula (III):
- M represents a hydrogen atom and/or a metallic cation from group (Ia) or (IIa), or an ammonium cation;
- Z₁, Z₂ and Z₃ have the meanings given in claims 2 to 4.

38. A process according to claim 36 or claim 37, **characterized in that** said acid is decarboxylated by adding a protonic acid of mineral origin, preferably hydrochloric acid or sulphuric acid or an organic acid to the reaction medium to obtain a pH of 3 or less.

39. A process according to one of claims 36 to 38, **characterized in that** the reaction medium is heated to a temperature between 120°C and 350°C, preferably between 150°C and 220°C and following cooling, the 4-hydroxybenzaldehyde which preferably has formula (IV) is separated.

40. A process according to claim 39, **characterized in that** the phenolic compound which preferably has formula (I) obtained following decarboxylation is recycled.

41. A process for preparing vanillin according to one of claims 1 to 40, **characterised in that** a mixture of phenolic compounds, o-hydroxymethylguaiacol (A) and p-hydroxymethylguaiacol (B), is selectively oxidised at the hydroxymethyl group in the 2 position of compound (A) to a carboxy group, and the hydroxymethyl group in the 4 position of compound (B) is selectively oxidised to a formyl group, thus producing a mixture of a 2-hydroxy-3-methoxybenzoic acid and vanillin, and the latter is then recovered.

42. A process for preparing vanillin according to one of claims 1 to 40, **characterised in that** a mixture of phenolic compounds, o-formylguaiacol (A) and p-formylguaiacol (B), is selectively oxidised at the formyl group in the 2 position of compound (A) to a carboxy group, thus producing a mixture of a 2-hydroxy-3-methoxybenzoic acid and vanillin, and the latter is then recovered.

43. A process for preparing ethylvanillin according to one of claims 1 to 40, **characterised in that** a mixture of phenolic compounds, o-hydroxymethylguetol (A) and p-hydroxymethylguetol (B), is selectively oxidised at the hydroxymethyl group in the 2 position of compound (A) to a carboxy group, and the hydroxymethyl group in the 4 position of compound (B) is selectively oxidised to a formyl group, thus producing a mixture of a 2-hydroxy-3-ethoxybenzoic acid and ethylvanillin, and the latter is then recovered.

44. A process for preparing ethylvanillin according to one of claims 1 to 40, **characterised in that** a mixture of phenolic compounds, o-formylguetol (A) and p-formylguetol (B), is selectively oxidised at the formyl group in the 2 position of compound (A) to a carboxy group, thus producing a mixture of a 2-hydroxy-3-ethoxybenzoic acid and ethylvanillin, and the latter is then recovered.

45. Use of the process described in one of claims 1 to 40 for preparing vanillin and ethylvanillin.
